# EUROPEAN PATENT SPECIFICATION

(11) **EP 1 709 023 B1**
(45) Date of publication and mention of the grant of the patent: **11.11.2009**
(21) Application number: 05704876.1
(22) Date of filing: 18.01.2005
(51) Int. Cl.: C07D 295/08, C07D 335/02, C07D 409/04, C07D 333/64, A61P 5/00

(54) **SELECTIVE ESTROGEN RECEPTOR MODULATORS**
SELEKTIVE ÖSTROGEN-REZEPTOR-MODULATOREN
MODULATEURS SELECTIFS DU RECEPTEUR DES OESTROGENES

(30) Priority: 22.01.2004 US 538303 P
(43) Date of publication of application: 11.10.2006
(73) Proprietor: ELI LILLY AND COMPANY, Indianapolis IN 46285 (US)
(72) Inventor: DODGE, Jeffrey, Alan, Indianapolis, Indiana 46256 (US); FRANK, Scott, Alan, Indianapolis, Indiana 46220 (US); HUMMEL, Conrad, Wilson, Louisville, Colorado 80027 (US)
(74) Representative: Burnside, Ivan John
(86) International application number: PCT/US2005/000022
(87) International publication number: WO 2005/073206

(56) References cited:
- EP-A- 0 729 951
- EP-A- 0 731 100
- EP-A- 0 761 659
- WO-A-95/10513

## Description

### Field of Invention

The present invention is in the field of medicine, particularly in the treatment of gynecological disorders. More specifically, the present invention relates to selective estrogen receptor modulators useful to treat endometriosis and uterine leiomyoma.

### Background of the Invention

Uterine leiomyoma/leiomyomata (uterine fibroid disease) is an old and ever present clinical problem that goes under a variety of names, including uterine fibrosis, uterine hypertrophy, uterine lieomyomata, myometrial hypertrophy, fibrosis uteri, and fibrotic metritis. Essentially, uterine fibrosis is a condition where there is an inappropriate deposition of fibroid tissue on the wall of the uterus. This condition is a cause of dysmenorrhea and infertility in women.

Endometriosis is a condition of severe dysmenorrhea, which is accompanied by severe pain, bleeding into the endometrial masses or peritoneal cavity and often leads to infertility. The symptom's cause appears to be ectopic endometrial growths that respond inappropriately to normal hormonal control and are located in inappropriate tissues. Because of the inappropriate locations for endometrial growth, the tissue seems to initiate local inflammatory-like responses causing macrophage infiltration and a cascade of events leading to initiation of the painful response. Evidence suggests that a cause of uterine fibrosis and endometriosis is an inappropriate response of fibroid tissue and/or endometrial tissue to estrogen.

Many publications have appeared within the last ten years disclosing novel selective estrogen receptor modulators (SERMs), *e*.*g*., U.S. Patent No.'s 5,484,795, 5,484,798, 5,510,358, 5,998,401 and WO 96/09040. Many of these SERMs, generally speaking, have been found to have a beneficial estrogen agonist activity in the bone and cardiovascular systems with a concomitant beneficial estrogen antagonist activity in the breast. A small, particularly useful subset of such compounds has also been found to have an estrogen antagonist effect in the uterus. A compound with this particularly useful SERM profile holds particular promise in treating uterine leiomyoma/leiomyomata and/or endometriosis.

EP 0761659 is directed to naphthyl and dihydronaphthyl compounds for the treatment of various medical indications associated with postmenopausal syndrome. WO 95/10513 is directed to benzothiophenes and related compounds as estrogen agonists.

However, the actual use of these SERM compounds, particularly in premenopausal women, has been hampered due to said compound's stimulatory effect on the ovaries. A great need currently exists, therefore, for new SERM compounds that behave as estrogen antagonists in the uterus that do not stimulate the ovaries.

### Summary of Invention

The present invention relates to a compound of formula I: 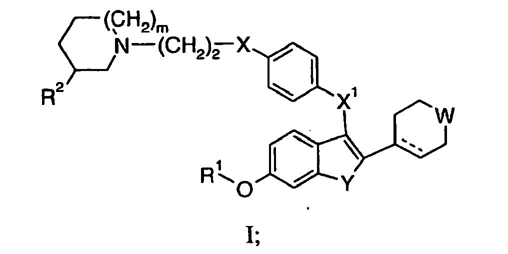 wherein:
m is 0, 1 or 2;
R¹ is H, SO₂(n-C₄-C₆ alkyl) or COR³;
R² is H or methyl provided that if m is 1 or 2, then R² must be H and that if m is 0, then R² must be methyl;
W is CHSO₂R⁴ or SO₂;
X is O or NR⁵;
X¹ is O, CH₂, or CO;
Y is S or CH=CH;
the dashed line ( --- ) represents an optional double bond;
R³ is C₁-C₆ alkyl, C₁-C₆ alkoxy, NR⁶R⁷, phenoxy, or phenyl optionally substituted with halo;
R⁴ is C₁-C₆ alkyl, C₁-C₆ alkoxy, NR⁸R⁹, CF₃ or CH₂CF₃;
R⁵ is H or C₁-C₆ alkyl
R⁶, R⁷ and R⁸ are independently H, C₁-C₆ alkyl or phenyl; and
R⁹ is C₁-C₆ alkyl or phenyl; or a pharmaceutical acid addition salt thereof.

The present invention also relates to a pharmaceutical composition containing a compound of formula I, or a pharmaceutical acid addition salt thereof, and a pharmaceutical carrier. In another embodiment, the pharmaceutical composition of the present invention may be adapted for use in treating endometriosis and/or uterine leiomyoma.

The present invention also relates to the use of a compound of formula I, or a pharmaceutical acid addition salt thereof, for the manufacture of a medicament for treating endometriosis and/or uterine leiomyoma.

The present invention further relates to a compound of formula II: 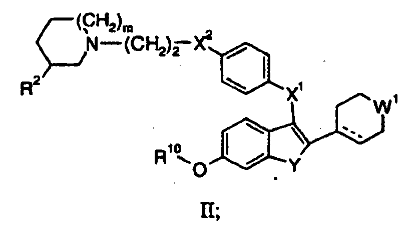 wherein:
m, R², R³, R⁴, X¹ and Y are as defined above for a formula I compound and
W¹ is CHS(O)ₙR⁴ or S(O)ₙ;
n is 0, 1 or 2;
R¹⁰ is H, C₁-C₆ alkyl, benzyl, SO₂CH₃, SO₂(n-C₄-C₆ alkyl) or COR⁴;
X² is O or NR¹¹; and
R¹¹ is H, C₁-C₆ alkyl or CO₂(C₁-C₆ alkyl); provided that if n is 2, then R¹⁰ is C₁-C₆ alkyl, SO₂CH₃ or benzyl or R¹¹ is CO₂(C₁-C₆ alkyl); or an acid addition salt thereof; useful as an intermediate to a compound of formula I.

### Detailed Description

Unless specified otherwise, reference hereafter to a "compound of formula I" includes the pharmaceutical acid addition salts thereof.

The compounds of the present invention have one or more chiral centers and may exist in a variety of stereoisomeric configurations. As a consequence of these chiral centers, the compounds of the present invention occur as racemates, mixtures of enantiomers and as individual enantiomers, as well as diastereomers and mixtures of diastereomers. All such racemates, enantiomers, and diastereomers are within the scope of the present invention.

For the purposes of the present invention, as disclosed and claimed herein, the following terms are defined below.

The term "halo" refers to fluoro, chloro, bromo and iodo. The term "C₁-C₆ alkyl" represents a straight, branched or cyclic hydrocarbon moiety having from one to six carbon atoms, *e*.*g*., methyl, ethyl, n-propyl, isopropyl, cyclopropyl, n-butyl, isobutyl, sec-butyl, t-butyl, cyclobutyl, pentyl, cyclopentyl, hexyl, cyclohexyl and the like. Moieties such as a cyclobutylmethylenyl are also included within the scope of a C₁-C₆ alkyl group. The term "C₁-C₄ alkyl" refers specifically to methyl, ethyl, n-propyl, isopropyl, cyclopropyl, n-butyl, isobutyl, sec-butyl, t-butyl and cyclobutyl. The term "n- C₄-C₆ alkyl" refers specifically to n-butyl, n-pentyl and n-hexyl. A "C₁-C₆ alkoxy" group is a C₁-C₆ alkyl moiety connected through an oxy linkage.

The term "pharmaceutical" when used herein as an adjective means substantially non-deleterious.

A pharmaceutical "acid addition salt" is a salt formed by reaction of the free base form of a compound of formula I with a pharmaceutical acid, such as described in the Encyclopedia of Pharmaceutical Technology, editors James Swarbrick and James C. Boylan, Vol 13, 1996 "Preservation of Pharmaceutical Products to Salt Forms of Drugs and Absorption". Specific salt forms include, but are not limited to the: acetate, benzoate, benzenesulfonate, 4-chlorobenzenesulfonate; citrate; ethanesulfonate; fumarate; d-gluconate; d-glucuronate; glutarate; glycolate; hippurate; hydrochloride; 2-hydroxyethanesulfonate; dl-lactate; maleate; d-malate; l-malate; malonate; d-mandelate; 1-mandelate; methanesulfonate; 1,5 napthalenedisulfonate; 2-naphthalenesulfonate; phosphate; salicylate; succinate; sulfate; d-tartrate; 1-tartrate; and p-toluenesulfonate.

The term "patient" as used herein refers to female humans and non-human female animals such as companion animals (dogs, cats, horses and the like).

The terms "treating" and "treat" as used herein means alleviating, ameliorating, preventing, prohibiting, restraining, slowing, stopping, or reversing the progression or severity of a pathological condition, or sequela thereof, described herein. The term "preventing" means reducing the likelihood that the recipient of a compound of formula I will incur, further incur or develop any of the pathological conditions, or sequela thereof, described herein.

The term "a patient in need thereof" is a patient either suffering from the caimed pathological condition or sequela thereof or is a patient at a recognized risk thereof as determined by medical diagnosis, *i*.*e*., as determined by the attending physician.

As used herein, the term "effective amount" means an amount of a compound of formula I that is capable of treating the conditions described herein.

### Preferred Compounds and Embodiments of the Invention

Certain compounds of the invention are particularly interesting and are preferred. The following listing sets out several groups of preferred compounds. It will be understood that each of the listings may be combined with other listings to create additional groups of preferred compounds.
a) m is 1 or 2;
b) m is 1;
c) R¹ is H;
d) R¹ is H or COR³ and R³ is C₁-C₆ alkyl, NHCH₃ or phenyl;
e) R¹ is H or COR³ and R³ is C₁-C₄ alkyl, NHCH₃ or phenyl;
f) R⁴ is C₁-C₄ alkyl, NR⁸R⁹ or CF₃ and R⁸ is H or C₁-C₄ alkyl and R⁹ is C₁-C₄ alkyl;
g) R⁴ is methyl, ethyl, cyclopropyl, NHCH₃, N(CH₃)₂ or CF₃;
h) R⁴ is methyl or N(CH₃)₂;
i) R⁴ is methyl;
j) R⁴ is N(CH₃)₂;
k) X is O;
l) X is NR⁵ and R⁵ is H or methyl;
m) X¹ is O or CH₂;
n) X¹ is O;
o) Y is S;
p) Y is CH=CH;
q) W is CHSO₂R⁴;
r) W is SO₂;
s) the optional double bond is not present and W is SO₂;
t) the optional double bond is not present and W is CHSO₂R⁴;
u) the optional double bond is present and W is CHSO₂R⁴;
v) the hydrochloride salt form.

The preferred patient of treatment is a female human.

The compound of formula I is preferably formulated in a dosage unit form, *i*.*e*., in an individual delivery vehicle, for example, a tablet or capsule, prior to administration to the recipient woman.

The compound of formula I is preferably administered orally.

### Synthesis

The compound of formula I may be prepared as described in the following Schemes and Examples.

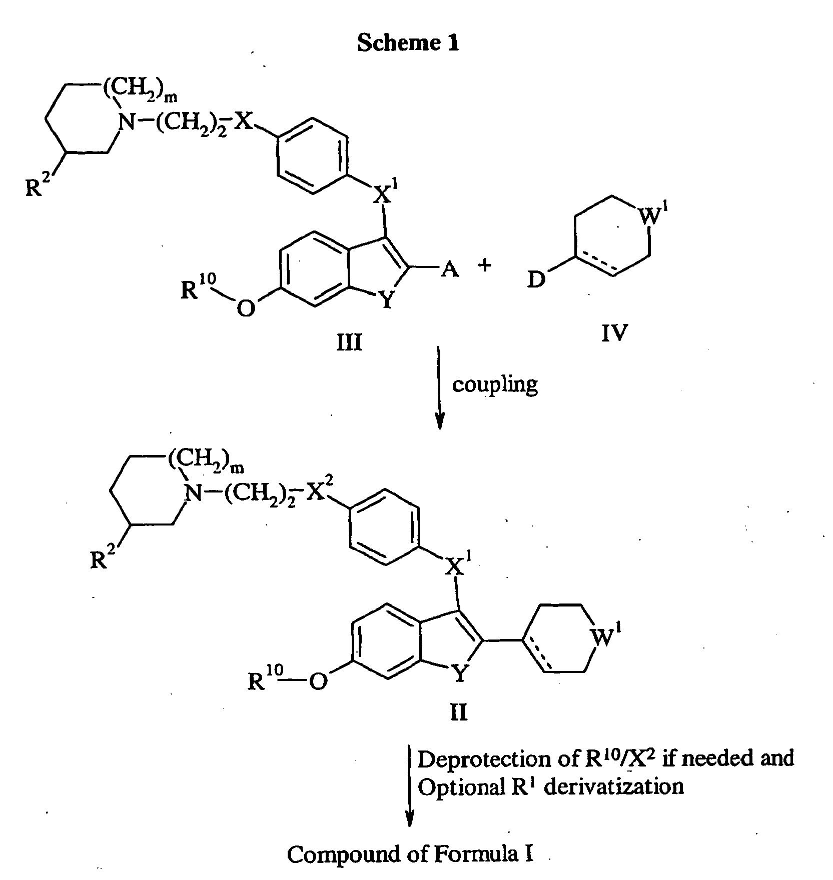

In Scheme 1, a compound of formula III is reacted with a compound of formula IV under usual "Suzuki" or "Stille" reaction conditions, *i*.*e*., wherein one of substituent "A" or "D" is a boronic acid/ester or alkyl stannane moiety and the other is a leaving group, *e*.*g*., chloro, bromo or iodo or a sulfonate group such as trifluoromethyl sulfonate to give the compound of formula II. When R¹⁰ is is SO₂CH₃, C₁-C₆ alkyl or benzyl (preferably methyl, benzyl or SO₂CH₃) said hydroxy protecting groups may be removed under standard conditions (see, *e*.*g*., the procedures that follow or the latest edition of Greene, Protective Groups in Organic Synthesis, John Wiley & Sons, New York, N.Y.) to provide the compound of formula I where R¹ is H. Similarly, when X² is NR¹¹ and R¹¹ is CO₂(C₁-C₆ alkyl), said amino protecting group may also be removed as taught in Greene. A formula I compound where R¹ is H may be further derivatized employing standard acylation or sulfonylation methodology to prepare a compound of formula I where R¹ is COR³ or SO₂(n-C₄-C₆ alkyl). In addition, when n is 0 or 1, the compound of formula II may be oxidized to the corresponding sulfone under standard conditions. Similarly, when the optional double bond is present in a compound of formula I or II, said double bond may be reduced under standard conditions.

### General Experimental Details

Electrospray mass spectra may be obtained on a Finnigan LCQ Duo instrument using a mobile phase of 50% acetonitrile, 25% methanol, and 25% 2mM aqueous ammonium acetate. Preparative HPLC's may be obtained on a Gilson Preparative System with Unipoint Software and dual wavelength detection at 220 and 254 nm as well as Finnigan aQa MS. A 20-mm x 250-mm ODS-AQ column with a particle size of 15 microns may be used as the stationary phase. The eluent is a binary system of bottle A (0.1% trifluoroacetic acid (TFA), 1% isopropyl alcohol (IPA) in water) and bottle B (0.05% TFA, 1% IPA in acetonitrile). The standard method is a gradient of 30-95% B unless otherwise indicated. The compounds purified by this method are isolated as TFA salts.

Preparative HPLC's may also be obtained on a Biotage ParallelFlex system with proprietary dual wavelength detection and software. A 30-mm x 150-mm or 19-mm x 250 mm Xterra column with a particle size of 10 microns is used as the stationary phase and 10mM NH₄⁺HCOO⁻/10mM NH₄OH is used as mobile phase A and 100% acetonitrile is used as a mobile phase B.

### Preparation 1

### Trifluoromethanesulfonic acid 6-methoxy-1-[4-(2-piperidin-1-yl-ethoxy)-phenoxy]-naphthalen-2-yl ester

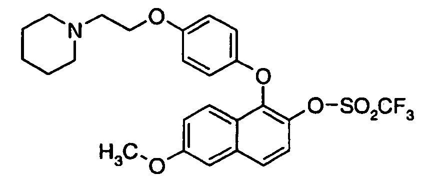

Add 6-methoxynaphthalene-2-ol (20 g, 114.8 mmol) to dimethylformamide (DMF, 250 mL) at ambient temperature followed by *N*-bromosuccinimide (NBS, 21.5 g, 120 mmol) over a 30-minute period. After 45 minutes, dilute with water (800 mL), collect and dry the precipitate to provide 25.5 g (87%) of 1-bromo-6-methoxy-naphthalen-2-ol.

Add 1-bromo-6-methoxy-naphthalen-2-ol (66.7 g, 264 mmol), potassium carbonate (K₂CO₃, 40.0 g, 290 mmol) and benzyl bromide (49.6 g, 290 mmol) to DMF (800 mL). Stir the mixture at ambient temperature for 1 hour. Add water (400 mL) to precipitate the product. Collect the precipitate and wash the filter cake with heptane (3X 125 mL) then dry to provide 83.7 g of 2-benzyloxy-1-bromo-6-methoxy-naphthalene (86.2%).

Combine toluene (200 mL), 2-benzyloxy-1-bromo-6-methoxy-naphthalene (30 g, 87.4 mmol), 4-(2-piperidin-1-yl-ethoxy)phenol (23.2 g, 105 mmol) and cesium carbonate (34.4 g, 105 mmol), heat the mixture to reflux. Remove a portion of the toluene (100 mL). Add ethyl acetate (390 mg, 4.37 mmol) and copper triflate benzene complex (2.20 g, 4.37 mmol) to the reaction mixture and stir for 5 minutes. Remove the solvent by distillation and heat the resulting residue to 174°C for 1.5 hours. Dissolve the residue in a mixture of ethyl acetate (200 mL) and aqueous hydrochloric acid (1 N, 90 mL). Separate and concentrate the organics to a residue. Column chromatograph the residue to give 12.4 g of 1-{2-[4-(2-benzyloxy-6-methoxy-naphthalen-1-yloxy)-phenoxy]-ethyl}-piperidine (30%).

Add 1-{2-[4-(2-benzyloxy-6-methoxy-naphthalen-1-yloxy)-phenoxy]-ethyl}-piperidine (12.4 g, 25.5 mmol) to a methanol/ethyl acetate mixture (1:1, 490 mL) and heat to form a solution. Remove the heat and add ammonium formate (4.83 g, 76.6 mmol) and Pd(OH)₂ on Carbon (20 % ww, 1.58 g, 1.12 mmol). Reflux for 50 minutes then filter the mixture. Concentrate the filtrate to provide 9.9 g of 6-methoxy-1-[4-(2-piperidin-1-yl-ethoxy)-phenoxy]-naphthalene-2-ol (98.5%).

Cool dichloromethane (290 mL), triethylamine (3.08 g, 30.4 mmol) and 6-methoxy-1-[4-(2-piperidin-1-yl-ethoxy)-phenoxy]-naphthalene-2-ol (9.2 g, 23.4 g) to -50°C and add trifluoromethane sulfonic acid anhydride (7.26 g, 25.7 mmol). Stir the resulting mixture at -50°C for 2 hours then allow the mixture to warm to ambient temperature before stirring for an additional hour. Add brine (150 mL) and separate the organics. Wash the organics with NaHCO₃ then dry before concentrating to a residue. Crystallize the residue with ethyl ether - hexanes to provide 11.2 g of the title compound (90.9%).

### Preparation 2

### (+/-)-2-(4-Methanesulfonyl-cyclohex-1-enyl)-4,4,5,5-tetramethyl-[1,3,2]dioxaborolane

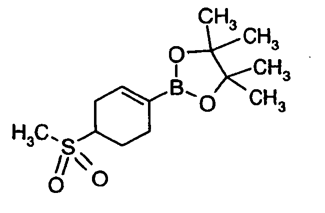

Combine vinyl sulfone (5 mL, 28.9 mmol), 2-trimethylsilyloxy-butadiene (2.8 g, 31.7 mmol), and toluene (25 mL) in a 50 mL round bottom flask fitted with a reflux condenser. Heat the reaction to reflux for 48 hours. Cool to ambient temperature and concentrate *in vacuo*. Dilute with dichloromethane (50 mL) and filter through Celite and concentrate *in vacuo*. Dissolve in methanol (5 mL) and trifluoroacetic acid (2 mL) and stir at ambient temperature for 2 hours. Concentrate *in vacuo*. Purify the residue by column chromatography using a silica gel column eluting with ethyl acetate. Isolate 1.0 g (26%) of 4-methanesulfonyl-cyclohexanone after concentrating the fractions.

Combine 4-methanesulfonyl-cyclohexanone (1.0 g, 5.7 mmol), 2,6-di-*t*-butyl-4-methylpyridine (2.6 g, 12.5 mmol), and dichloromethane (10 mL) in a 25 mL round bottom flask fitted with a reflux condenser. Add triflic anhydride (1.9 mL, 11.3 mmol). Heat the reaction to reflux for 12 hours. Cool to ambient temperature and pour into ether (150 mL). Filter and concentrate *in vacuo.* Purify the residue by column chromatography using a silica gel column eluting with a linear gradient beginning with hexanes and ending with 2:5 hexanes:ethyl acetate. Isolate 1.4 g (82%) of (+/-)-trifluoro-methanesulfonic acid 4-methanesulfonyl-cyclohex-1-enyl ester after concentrating the fractions.

Combine (+/-)-trifluoro-methanesulfonic acid 4-methanesulfonyl-cyclohex-1-enyl ester (500 mg, 1.6 mmol), bis(pinacolotodiboron) (450 mg, 1.8 mmol) and dimethylsulfoxide (5 mL). Bubble nitrogen through this solution for 15 minutes. Add potassium acetate (480 mg, 4.9 mmol) and dichloro[1,1'-bis(diphenylphosphino)ferrocene]palladium dichloromethane adduct (120 mg, 0.16 mmol). Heat the mixture to 70°C for 12 hours. Cool to ambient temperature. Partition between ether (50 mL) and saturated aqueous brine (15 mL). Extract the organic component. Dry over magnesium sulfate, filter, and concentrate *in vacaco*. Purify the residue by column chromatography using a 5% triethylamine/hexanes prewashed silica gel column eluting with 5:1 ethyl acetate:hexanes. Isolate 400 mg (86%) of (+/-)-2-(4-methanesulfonyl-cyclohex-1-enyl)-4,4,5,5-tetramethyl-[1,3,2]dioxaborolane after concentrating the fractions.

### Example 1

### (+/-)-1-(2-{4-[2-(4-Methanesulfonyl-cyclohex-1-enyl)-6-methoxy-naphthalen-1-yloxy]-phenoxy}-ethyl)-piperidine Hydrochloride

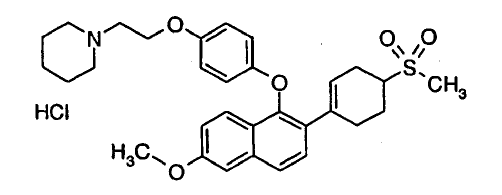

Combine trifluoro-methanesulfonic acid 6-methoxy-1-[4-(2-piperidin-1-yl-ethoxy)-phenoxy]-naphthalen-2-yl ester (910 mg, 1.7 mmol) and (+/-)-2-(4-methanesulfonyl-cyclohex-1-enyl)-4,4,5,5-tetramethyl-[1,3,2]dioxaborolane (990 mg, 3.5 mmol) in acetonitrile (20 mL). Bubble nitrogen through the solution for 10 minutes. Add palladium(n) acetate (39 mgs, 0.2 mmol), tricyclohexylphosphine (0.5 mL, 0.3 mmol, 20% solution in toluene), and cesium fluoride (2.4 g, 15.6 mmol). Fit the flask with a reflux condenser and heat to reflux for 30 minutes. Cool to ambient temperature and dilute with dichloromethane (50 mL) and saturated aqueous ammonium chloride (10 mL). Separate the organic and wash the aqueous twice with dichloromethane (10 mL). Combine the organics and dry over magnesium sulfate. Filter and concentrate *in vacuo*. Purify the residue by column chromatography using a silica gel column eluting with a linear gradient beginning with dichloromethane and ending with 5:1 dichloromethane:methanol. Isolate 900 mg (95%) of (+/-)-1-(2-{4-[2-(4-methanesulfonyl-cyclohex-1-enyl)-6-methoxy-naphthalen-1-yloxy]-phenoxy}-ethyl)-piperidine after concentrating the fractions: mass spectrum (ion spray): m/z = 536.2 (M+H).

Dissolve (+/-)-1-(2-{4-[2-(4-methanesulfonyl-cyclohex-1-enyl)-6-methoxy-naphthalen-1-yloxy]-phenoxy}-ethyl)-piperidine (500 mg, 0.93 mmol) in CH₂Cl₂ (20 mL). Add hydrogen chloride (1.2 mL, 1.0 M in ether) and stir the reaction mixture for 10 minutes. Concentrate *in vacuo*. Isolate the title compound, 530 mg (100%): mass spectrum (ion spray): m/z = 536.2 (M+H-HCl).

### Example 2

### (+/-)-6-(4-Methanesulfonyl-cyclohex-1-enyl)-5-[4-(2-piperidin-1-yl-ethoxy)-phenoxy]-naphthalen-2-ol Hydrochloride

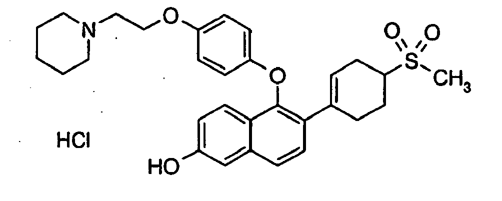

Dissolve (+/-)-1-(2-{4-[2-(4-Methanesulfonyl-cyclohex-1-enyl)-6-methoxy-naphthalen-1-yloxy]-phenoxy}-ethyl)-piperidine Hydrochloride (530 mg, 0.93 mmol) in CH₂C1₂ (20 mL) and 2-methyl-1-butene (5 mL). Cool the solution to 0°C and add BBr₃ (0.3 mL, 3.3 mmol). Allow to warm to ambient temperature over 1 hour. Add methanol (10 mL) and stir 30 minutes. Add silica gel (5 g) and concentrate *in vacuo*. Purify the residue by column chromatography using a silica gel column eluting with a linear gradient beginning with dichloromethane and ending with 5:1 dichloromethane:methanol. Isolate 400 mg (82%) of (+/-)-6-(4-methanesulfonyl-cyclohex-1-enyl)-5-[4-(2-piperidin-1-yl-ethoxy)-phenoxy]-naphthalen-2-ol after concentrating the fractions: mass spectrum (ion spray): m/z = 522.2 (M+H).

Dissolve (+/-)-6-(4-methanesulfonyl-cyclohex-1-enyl)-5-[4-(2-piperidin-1-yl-ethoxy)-phenoxy]-naphthalen-2-ol (400 mg, 0.77 mmol) in CH₂Cl₂ (5 mL). Add hydrogen chloride (1 mL, 1.0 M in ether) and stir the reaction mixture for 10 minutes. Concentrate *in vacuo.* Stir the residue with ethanol (10 mL). Filter and dry the solid *in vacuo* to give 290 mg (60%) of the title compound: mass spectrum (ion spray): m/z = 522.2 (M+H-HCl).

### Examples 3. 4 and 5

### 6-cis-and-trans-(4-Methanesulfonyl-cyclohexyl)-5-[4-(2-piperidin-1-yl-ethoxy)-phenoxy]-naphthalen-2-ol Hydrochloride and a Mixture thereof

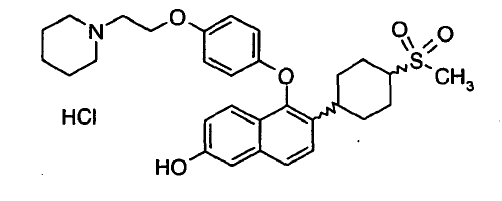

Dissolve (+/-)-1-(2-{4-[2-(4-methanesulfonyl-cyclohex-1-enyl)-6-methoxy-naphthalen-1-yloxy]-phenoxy}-ethyl)-piperidine (250 mg, 0.48 mmol) in tetrahydrofuran (25 mL) and ethanol (25 mL). Add palladium black (100 mg) and pressurize the reaction vessel with hydrogen (50 psi). Heat the reaction to 50°C for 12 hours. Cool to ambient temperature and filter through Celite, rinsing the pad with tetrahydrofuran (100 mL). Concentrate *in vacuo.* Redissolve the residue in dichloromethane (20 mL) and treat with hydrogen chloride (1 mL, 1.0 M in ether). Concentrate *in vacuo.* Redissolve the residue in dichloromethane (20 mL) and cool to 0°C. Add boron tribromide (0.15 mL, 1.6 mmol) and warm to ambient temperature for 1 hour. Add methanol (10 mL) and concentrate in *vacuo* in the presence of silica gel (5 g). Purify the residue by column chromatography using a silica gel column eluting with a linear gradient beginning with dichloromethane and ending with 5:1 dichloromethane:methanol. Combine the fractions containing product and concentrate *in* vacuo. Redissolve the residue in dichloromethane (20 mL) and treat with hydrogen chloride (1 mL, 1.0 M in ether). Dry the solid overnight *in* vacuo to give 160 mg (61%) of the title compounds as a 2:1 mixture of *trans*:*cis* isomers: mass spectrum (ion spray): m/z = 524.2 (M+H-HCl). (Example 3)

Separate the individual isomers (20 mg of the mixture) by chiral chromatography (Chiralpak AD column) using a 3:2 ethanol:hexanes with 0.2% dimethylethylamine eluent. Individually dissolve the residues in dichloromethane (2 mL) and treat with hydrogen chloride (0.1 mL, 1.0 M in ether). Dry the solids overnight *in* vacuo. Isolate 6-*trans*-(4-methanesulfonyl-cyclohexyl)-5-[4-(2-piperidin-1-yl-ethoxy)-phenoxy]-naphthalen-2-ol hydrochloride (9 mgs, Example 4) and 6- *cis*-(4-methanesulfonylcyclohexyl)-5-[4-(2-piperidin-1-yl-ethoxy)-phenoxy]-naphthalen-2-ol hydrochloride (7 mgs, Example 5).

### Preparation 3

### 2-(3,6-Dihydro-2H-thiopyran-4-yl)-4,4,5,5-tetramethyl-[1,3,2]dioxaborolane

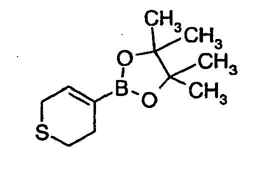

Combine diisopropylamine (2.5 mL, 18.1 mmol) and tetrahydrofuran (25 mL) and cool to 0°C. Add *n*-butyllithium (11 mL, 18.1 mmol, 1.6 M in hexanes) dropwise over 15 minutes. Cool to -78°C. Add tetrahydro-thiopyran-4-one (2 g, 17.2 mmol) dropwise over 20 minutes as a solution in tetrahydrofuran (20 mL). Stir at -78°C for 20 minutes. Add N-phenylbis(trifluoromethanesulfonimide) (6.8 g, 18.9 mmol) from a powder addition funnel and rinse into the reaction with tetrahydrofuran (5 mL). Allow to warm to ambient temperature and stir overnight. Partition between ether (100 mL) and 1 M aqueous sodium hydroxide (25 mL). Separate organic and wash with 1 M aqueous sodium bisulfate (25 mL) and finally with saturated aqueous brine. Dry the organic over sodium sulfate, decant, and concentrate *in vacuo*. Purify the residue by column chromatography using a silica gel column eluting with a linear gradient beginning with hexanes and ending with 5:1 hexanes:ethyl acetate. Isolate 3.5 g (81%) of trifluoro-methanesulfonic acid 3,6-dihydro-2H-thiopyran-4-yl ester after concentrating the fractions.

Combine trifluoro-methanesulfonic acid 3,6-dihydro-2H-thiopyran-4-yl ester (1 g, 4.0 mmol), bis(pinacolotodiboron) (1.1 g, 4.4 mmol) and dimethylsulfoxide (15 mL). Bubble nitrogen through this solution for 15 minutes. Add potassium acetate (1.2 g, 12 mmol) and dichloro[1,1'-bis(diphenylphosphino)ferrocene]palladium dichloromethane adduct (290 mg, 0.40 mmol). Heat the mixture to 70°C for 12 hours. Cool to ambient temperature. Partition between ether (100 mL) and water (25 mL). Extract the organic component. Wash the organic with saturated aqueous brine (25 mL). Dry over sodium sulfate, decant, and concentrate *in vacuo*. Purify the residue by column chromatography using a 5% triethylamine/hexanes prewashed silica gel column eluting with 1:1 ether:hexanes to give 900 mg (100%) of 2-(3,6-dihydro-2H-thiopyran-4-yl)-4,4,5,5-tetramethyl-[1,3,2]dioxaborolane after concentrating the fractions.

### Example 6

### 1-(2-{4-[2-(1,1-Dioxo-1,2,3,6-tetrahydro-1λ⁶-thiopyran-4-yl)-6-methoxy-naphthalen-1-yloxy]-phenoxy}-ethyl)-piperidine

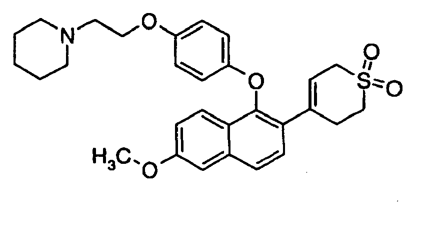

Combine trifluoromethanesulfonic acid 6-methoxy-1-[4-(2-piperidin-1-yl-ethoxy)-phenoxy]-naphthalen-2-yl ester (780 mg, 1.5 mmol) and 2-(3,6-dihydro-2H-thiopyran-4-yl)-4,4,5,5-tetramethyl-[1,3,2]dioxaborolane (700 mg, 3.0 mmol) in acetonitrile (25 mL). Bubble nitrogen through the solution for 10 minutes. Add palladium(II) acetate (34 mg, 0.15 mmol), tricyclohexylphosphine (0.36 mL, 0.23 mmol, 20% solution in toluene), and cesium fluoride (2.0 g, 13.3 mmol). Fit the flask with a reflux condenser and heat to reflux for 4 hours. Cool to ambient temperature and dilute with dichloromethane (50 mL) and saturated aqueous ammonium chloride (10 mL). Separate the organic and wash the aqueous twice with dichloromethane (10 mL). Combine the organics and dry over magnesium sulfate. Filter and concentrate *in vacuo*. Purify the residue by column chromatography using a silica gel column eluting with a linear gradient beginning with dichloromethane and ending with 5:1 dichloromethane:methanol to give 650 mg (90%) of 1-(2-{4-[2-(3,6-dihydro-2H-thiopyran-4-yl)-6-methoxy-naphthalen-1-yloxy]-phenoxy}-ethyl)-piperidine after concentrating the fractions.

Redissolve in methanol (10 mL) and dichloromethane (20 mL). Add water (8 mL) and oxone (2.0 g, 3.4 mmol). Stir at ambient temperature for 4 hours. Dilute with dichloromethane (50 mL) and saturated aqueous sodium bicarbonate. Separate the organic, dry over magnesium sulfate, filter and concentrate *in vacuo*. Purify the residue by column chromatography using a silica gel column eluting with a linear gradient beginning with dichloromethane and ending with 5:1 dichloromethane:methanol to give 370 mg (53%) of the title compound after concentrating the fractions: mass spectrum (ion spray): m/z = 508.1 (M+H).

### Example 7

### 1-(2-{4-[2-(1,1-Dioxo-1,2,3,6-tetrahydro-1λ⁶-thiopyran-4-yl)-6-methoxy-naphthalen-1-yloxy]-phenoxy}-ethyl)-piperidine Hydrochloride

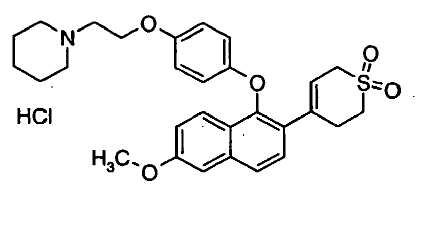

Dissolve 1-(2-{4-[2-(1,1-dioxo-1,2,3,6-tetrahydro-1λ⁶-thiopyran-4-yl)-6-methoxy-naphthalen-1-yloxy]-phenoxy}-ethyl)-piperidine (400 mg, 0.77 mmol) in CH₂Cl₂ (5 mL). Add hydrogen chloride (1 mL, 1.0 M in ether) and stir the reaction mixture for 10 minutes. Concentrate *in vacuo* to give the title compound, 390 mg (100%): mass spectrum (ion spray): m/z = 508.1 (M+H-HCl).

### Example 8

### 6-(1,1-Dioxo-1,2,3,6-tetrahydro-1λ⁶-thiopyran-4-yl)-5-[4-(2-piperidin-1-yl-ethoxy)-phenoxy]-naphthalen-2-ol

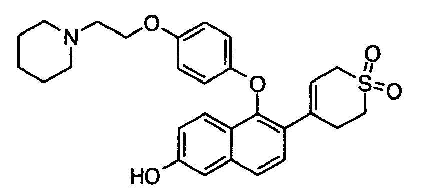

Dissolve 1-(2-{4-[2-(1,1-dioxo-1,2,3,6-tetrahydro-1λ⁶-thiopyran-4-yl)-6-methoxy-naphthalen-1-yloxy]-phenoxy}-ethyl)-piperidine hydrochloride (370 mg, 0.72 mmol) in CH₂Cl₂ (10 mL) and 2-methyl-1-butene (2 mL). Cool the solution to 0°C and add BBr₃ (0.24 mL, 2.5 mmol). Allow to warm to ambient temperature over 1 hour. Add methanol (10 mL) and stir 30 minutes. Add silica gel (5 g) and concentrate *in vacuo*. Purify the residue by column chromatography using a silica gel column eluting with a linear gradient beginning with dichloromethane and ending with 5:1 dichloromethane:methanol to give 290 mg (82%) of the title compound after concentrating the fractions: mass spectrum (ion spray): m/z = 494.1 (M+H).

### Example 9

### 6-(1,1-Dioxo-1,2,3,6-tetrahydro-1λ⁶-thiopyran-4-yl)-5-[4-(2-piperidin-1-yl-ethoxy)-phenoxy]-naphthalen-2-ol Hydrochloride

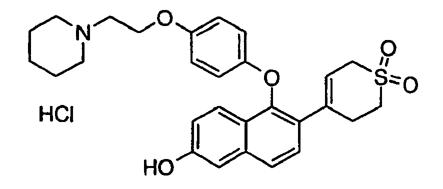

Dissolve 6-(1,1-dioxo-1,2,3,6-tetrahydro-1λ⁶-thiopyran-4-yl)-5-[4-(2-piperidin-1-yl-ethoxy)-phenoxy]-naphthalen-2-ol (290 mg, 0.59 mmol) in CH₂Cl₂ (5 mL). Add hydrogen chloride (1 mL, 1.0 M in ether) and stir the reaction mixture for 10 minutes. Concentrate *in vacuo*. Dissolve in methanol (5 mL) and add water (1.5 mL). Filter through decolorizing carbon and concentrate *in vacuo* to give 180 mg of the title compound (60%): mass spectrum (ion spray): m/z = 494.1 (M+H-HCl).

### Example 10

### 6-(1,1-Dioxo-hexahydro-1λ⁶-thiopyran-4-yl)-5-[4-(2-piperidin-1-yl-ethoxy)-phenoxy]-naphthalen-2-ol Hydrochloride

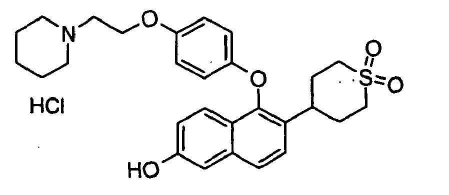

Dissolve 6-(1,1-dioxo-1,2,3,6-tetrahydro-1λ⁶-thiopyran-4-yl)-5-[4-(2-piperidin-1-yl-ethoxy)-phenoxy]-naphthalen-2-ol hydrochloride (100 mg, 0.2 mmol) in tetrahydrofuran (15 mL) and methanol (10 mL). Add palladium black (50 mgs) and pressurize the reaction vessel with hydrogen (55 psi). Heat the reaction to 60°C for 12 hours. Cool to ambient temperature and filter through Celite, rinsing the pad with tetrahydrofuran (100 mL). Concentrate *in vacuo*. Redissolve the residue in dichloromethane (20 mL) and treat with hydrogen chloride (1 mL, 1.0 M in ether). Concentrate *in vacuo*. Redissolve the residue in dichloromethane (5 mL) and cool to 0°C. Add boron tribromide (75 µL, 1.6 mmol) and warm to ambient temperature for 1 hour. Add methanol (5 mL) and concentrate *in vacuo* in the presence of silica gel (5 g). Purify the residue by column chromatography using a silica gel column eluting with a linear gradient beginning with dichloromethane and ending with 5:1 dichloromethane:methanol. Combine the fractions containing product and concentrate *in* vacuo. Redissolve the residue in dichloromethane (5 mL) and treat with hydrogen chloride (1 mL, 1.0 M in ether). Dry the solid overnight *in* vacuo to give 40 mg (38%) of 6-(1,1-dioxo-hexahydro-1λ⁶-thiopyran-4-yl)-5-[4-(2-piperidin-1-yl-ethoxy)-phenoxy]-naphthalen-2-ol hydrochloride: mass spectrum (ion spray): m/z = 496.1 (M+H-HCl).

### Preparation 4

### 4-{6-Methoxy-3-[4-(2-piperidin-1-yl-ethoxy)-phenoxy]-benzo[b]thiophen-2-yl}-tetrahydro-thiopyran-4-ol

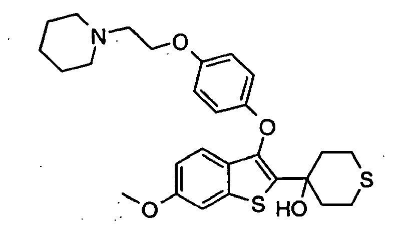

Dissolve 1-{2-[4-(2-bromo-6-methoxy-1,1-dioxo-1H-1λ⁶-benzo[b]thiophen-3-yloxy)-phenoxy]-ethyl}-piperidine (3.0 g, 6 mmol) in tetrahydrofuran (20 mL). Add methanol (30 mL). Add 10% palladium/carbon (0.34 g). Evacuate reaction vessel. Backfill with hydrogen. Repeat twice. Stir reaction mixture overnight. Evacuate reaction vessel. Backfill with nitrogen. Filter through Celite, rinsing with tetrahydrofuran (50 mL). Concentrate *in vacuo*. Purify the residue by column chromatography using a silica gel column eluting with a linear gradient beginning with dichloromethane and ending with 5 : 1 dichloromethane : methanol. Recrystallize the residue from ethanol to give 2.4 g (96%) of 1-{2-[4-(6-methoxy-1,1-dioxo-1H-1λ6-benzo[b]thiophen-3-yloxy)-phenoxy]-ethyl}-piperidine.

Dissolve 1-{2-[4-(6-methoxy-1,1-dioxo-1H-1λ6-benzo[b]thiophen-3-yloxy)-phenoxy]-ethyl}-piperidine (14.4 g, 34.7 mmol) in dioxane (200 mL). Add diisobutylaluminum hydride (30 mL, 168 mmol) and heat the solution to reflux for 2 h. Cool to room temperature and then to -78 °C. Add ethyl acetate (50 mL) and warm to room temperature. Pour the reaction into 10% aqueous solution of sodium potassium tartrate. Dilute with ethyl acetate (500 mL). Stir overnight at room temperature. Transfer to a separatory funnel. Extract the organic layer. Wash the aqueous layer with ethyl acetate (100 mL). Combine the organics and wash with saturated aqueous brine. Dry over sodium sulfate, decant, and concentrate *in vacuo*. Purify the residue by column chromatography using a silica gel column eluting with a linear gradient beginning with dichloromethane and ending with 7 : 1 dichloromethane : methanol to give 13.0 g (98%) of 1-{2-[4-(6-methoxy-benzo[b]thiophen-3-yloxy)-phenoxy]-ethyl}-piperidine.

Dissolve 1-{2-[4-(6-methoxy-benzo[b]thiophen-3-yloxy)-phenoxy]-ethyl}-piperidine (1.0 g, 2.6 mmol) in tetrahydrofuran (30 mL) and cool to -78 °C. Add *n*-butyl lithium (1.8 mL, 2. 8 mmol, 1.6 M in hexanes) and stir the reaction 15 minutes. Add tetrahydro-thiopyran-4-one (0.6 g, 5.2 mmol) as a solid and allow the reaction to stir overnight, warming to room temperature. Dilute with ethyl acetate (75 mL) and saturated aqueous ammonium chloride (25 mL). Separate the organic and wash with saturated aqueous sodium chloride. Dry over magnesium sulfate, filter, and concentrate *in vacuo* to give 1.1 g (100%) of the title compound.

### Example 11 (comparative)

### 1-(2-{4-[6-Methoxy-2-(tetrahydro-thiopyran-4-yl)-benzo[b]thiophen-3-yloxy]-phenoxy}-ethyl)-piperidine

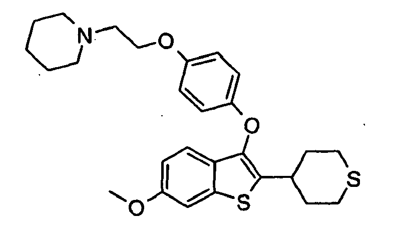

Dissolve 4-{6-methoxy-3-[4-(2-piperidin-1-yl-ethoxy)-phenoxy]-benzo[b]thiophen-2-yl}-tetrahydro-thiopyran-4-ol (1.1 g, 2.6 mmol) in dichloromethane (25 mL). Cool the solution to -78 °C and add trifluoroacetic acid (6 mL). Add sodium borohydride and allow the reaction to warm to room temperature. Partition the reaction between saturated ammonium chloride (50 mL) and dichloromethane (100 mL). Separate the organic layer and dry over magnesium sulfate. Filter and concentrate *in vacuo*. Purify the residue by column chromatography using a silica gel column eluting with a linear gradient beginning with dichloromethane and ending with 5 : 1 dichloromethane : methanol to give 0.84 g (67%) of the title compound: mass spectrum (ion spray) m/z = 484.3 (M+H).

### Example 12

### 3-[4-(2-Piperidin-1-yl-ethoxy)-phenoxy]-2-(tetrahydro-thiopyran-4-yl)-benzo[b]thiophen-6-ol

Dissolve 1-(2-{4-[6-methoxy-2-(tetrahydro-thiopyran-4-yl)-benzo[b]thiophen-3-yloxy]-phenoxy}-ethyl)-piperidine (0.84 g, 1.74 mmol) in dichloromethane (10 mL). Add 1.0 M hydrogen chloride in ether (2 mL, 2 mmol) and concentrate *in vacuo*. Dissolve in dichloromethane (25 mL) and cool to 0 °C. Add boron tribromide (0.5 mL, 5.21 mmol) and allow to warm to room temperature. Stir 2 h. Cool to 0 °C and add methanol (15 mL). Add silica gel (10 g) and concentrate *in vacuo*. Purify the residue by column chromatography using a silica gel column eluting with a linear gradient beginning with dichloromethane and ending with 5 : 1 dichloromethane : methanol to give 0.50 g (61%) of the title compound: mass spectrum (ion spray) m/z = 470.2 (M+H).

### Example 13

### 2-(1,1-Dioxo-hexahydro-1λ⁶-thiopyran-4-yl)-3-[4-(2-piperidin-1-yl-ethoxy)-phenoxy]-benzo[b]thiophen-6-ol hydrochloride

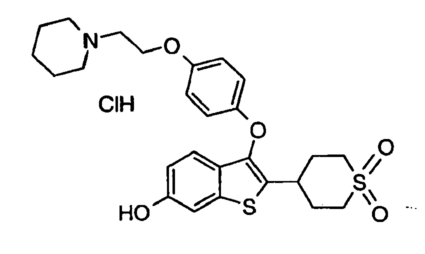

Dissolve 3-[4-(2-piperidin-1-yl-ethoxy)-phenoxy]-2-(tetrahydro-thiopyran-4-yl)-benzo[b]thiophen-6-ol (0.50 g, 1.1 mmol) in dichloromethane (20 mL). Add N-methylmorpholine N-oxide (0.31 g, 2.7 mmol) and osmium tetroxide (1.1 mL, 0.11 mmol, 0.1 M solution in toluene). Stir dark brown solution 2 hours. Dilute with dicloromethane (50 mL) and 10% aqueous sodium sulfite. Stir 10 minutes and separate organic. Wash with saturated aqueous sodium bicarbonate and dry over sodium sulfate. Decant and concentrate *in vacuo*. Purify the residue by column chromatography using a silica gel column eluting with a linear gradient beginning with dichloromethane and ending with 5 : 1 dichloromethane : methanol. Combine the product containing fractions and concentrate *in vacuo*. Dissolve the residue in ethyl acetate and add 1.0 M hydrogen chloride in ether (1 mL). Filter the solid dry *in vacuo* at 45 °C. Isolate 0.28 g of an off-white solid (53%): mass spectrum (ion spray) m/z = 502.2 (M-Cl).

### Examples 14 and 15

### 6-(4-Methanesulfonyl-cyclohex-1-enyl)-5-[4-(2-piperidin-1-yl-ethoxy)-phenoxy]-naphthalen-2-ol Isomers 1 and 2

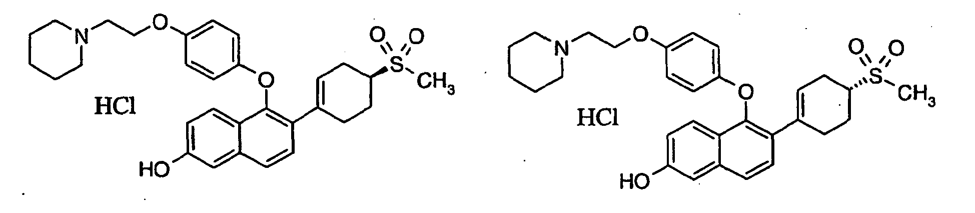

Combine 6-(4-Methanesulfonyl-cyclohex-1-enyl)-5-[4-(2-piperidin-1-yl-ethoxy)-phenoxy]-naphthalen-2-ol (1.0 g, 1.79 mmol), benzyl alcohol (0.39 mL, 3.76 mmol), and triphenylphosphine (0.99 g, 3.76 mmol) in CH₂Cl₂ (20 mL) and add diisopropyl azodicarboxylate (0.74 mL, 3.76 mmol) dropwise over 5 minutes. Allow the mixture to stir at ambient temperature for 16 hours. Transfer the reaction mixture to a 10 g SCX column using methanol. Wash the column with methanol (2x50 mL). Elute the product using 2M ammonia/ methanol (2x35 mL). Concentrate the eluent to obtain 1.1 g (100%) of racemic 1-(2-{4-[6-benzyloxy-2-(4-methanesulfonyl-cyclohex-1-enyl)-naphthalen-1-yloxy]-phenoxy}-ethyl)-piperidine. Resolve the enantiomers on a 4.6x150mm Chiralpak AD-H column eluting with 97/3 3A ethanol/acetonitrile with 0.2% N,N-Dimethyl ethylamine to obtain 350 mg (32%) of 1-(2-{4-[6-Benzyloxy-2-(4-methanesulfonyl-cyclohex-1-enyl)-naphthalen-1-yloxy]-phenoxy}-ethyl)-piperidine isomer 1 and 350 mg (32%) 1-(2-{4-[6-Benzyloxy-2-(4-methanesulfonyl-cyclohex-1-enyl)-naphthalen-1-yloxy]-phenoxy}-ethyl)-piperidine isomer 2.

Dissolve 1-(2-{4-[benzyloxy-2-(4-methanesulfonyl-cyclohex-1-enyl)-naphthalen-1-yloxy]-phenoxy}-ethyl)-piperidine isomer 1 (350 mg, 0.57 mmol) in CH₂Cl₂ (10 mL) and treat with hydrogen chloride (0.75 mL, 1.0 M in ether) and stir the reaction mixture for 10 minutes. Concentrate *in vacuo* to obtain 370 mg (99%) of 1-(2-{4-[6-benzyloxy-2-(4-methanesulfonyl-cyclohex-1-enyl)-naphthalen-1-yloxy]-phenoxy}-ethyl)-piperidine isomer 1 hydrochloride.

Dissolve 1-(2-{4-[6-benzyloxy-2-(4-methanesulfonyl-cyclohex-1-enyl)-naphthalen-1-yloxy]-phenoxy}-ethyl)-piperidine isomer 1 hydrochloride (370 mg, 0.58 mmol) in CH₂Cl₂ (20 mL) and 2-methyl-1-butene (3 mL). Cool the solution to 0°C and add BBr₃ (2.0 mL, 2.0 mmol, 1M in CH₂Cl₂). Stir at 0°C for 1 hour, then warm to ambient temperature and stir another 1 hour. Partition between CH₂Cl₂ (50 mL) and saturated aqueous sodium bicarbonate solution (20 mL). Separate the organic layer and wash with brine solution (30 mL), dry over magnesium sulfate, filter and concentrate in vacuo. Purify the residue by column chromatography using a silica gel column eluting with 1:1 hexane:ethyl acetate + 2% 7M ammonia/methanol to obtain 190 mg (62%) of 6-(4-methanesulfonyl-cyclohex-1-enyl)-5-[4-(2-piperidin-1-yl-ethoxy)-phenoxy]-naphthalen-2-ol isomer 1:mass spectrum (ion spray): m/z = 522.2 (M+H).

Dissolve 6-(4-methanesulfonyl-cyclohex-1-enyl)-5-[4-(2-piperidin-1-yl-ethoxy)-phenoxy]-naphthalen-2-ol isomer 1 (0.19 g, 0.36 mmol) in CH₂Cl₂ (10 mL) and treat with hydrogen chloride (0. 5 mL, 1.0 M in ether) and stir the reaction mixture for 10 minutes. Concentrate in vacuo to obtain 190 mg (95%) of the title compound as the pure enantiomer isomer 1: mass spectrum (ion spray): m/z = 522.2 (M+H-HCl).

Repeat the procedures above for 1-(2-{4-[6-benzyloxy-2-(4-methanesulfonyl-cyclohex-1-enyl)-naphthalen-1-yloxy]-phenoxy}-ethyl)-piperidine isomer 2 to obtain 370 mg (99%) of 1-(2-{4-[6-benzyloxy-2-(4-methanesulfonyl-cyclohex-1-enyl)-naphthalen-1-yloxy]-phenoxy}-ethyl)-piperidine hydrochloride isomer 2 and 180 mg of the title compound as the pure enantiomer 2: mass spectrum (ion spray): m/z = 522.2 (M+H-HCl).

### Preparation 5

### 6-Methoxy-3-[4-(2-piperidin-1-yl-ethoxy)-phenoxy]-2-bromobenzo[b]thiophene

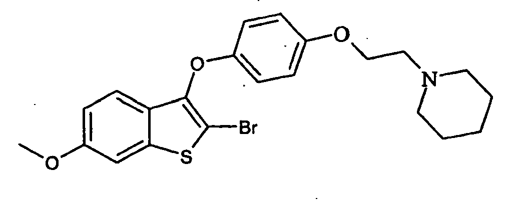

To a mixture of 3-methoxythiophenol and potassium carbonate in 850 mL of acetone is added dropwise bromoacetaldehyde diethyl acetal at room temperature. The heterogeneous mixture is stirred for 18 hrs and then filtered through a glass frit to remove salts. The filtered cake is washed (2 x 250 mL) with acetone and the filtrate is concentrated using a rotor evaporator. The filtrate is dissolved in diethyl ether (840 mL) and washed with water (850 mL), 1N NaOH (850 mL), and then brine (850 mL). The organic layer is dried over magnesium sulfate and concentrated using a rotor evaporator to give 212g of a crude intermediate.

A 12 L flask is charged with 51 mL of boron trifluoride etherate and dissolved in 7.6 L of dichloromethane. 100 g of the crude intermediate prepared above is dissolved in 771 mL of dichloromethane and placed in a 1 L addition funnel. This mixture is added dropwise over the course of 30-45 min. After the addition is complete, the mixture is stirred for an additional hour and then 1 L of sat. sodium bicarbonate is added. The mixture is stirred until both layers are clear. The aqueous layer is extracted with an additional 500 mL of dichloromethane. The combined organic solutions are dried over magnesium sulfate and concentrated under a rotor evaporator (63.1 g crude). The residue is purified by the following protocol: 250 mL of heptane is added to the mixture and stirred for 15 min. This mixture is filtered through a silica gel plug which is washed with heptane (5 x 250 mL) and concentrated (40.83 g). The residue is distilled under vacuum (148 °C/3 mm Hg) to provide 6-methoxybenzothiophene.

A 5 L flask is charged with 6-methoxybenzothiophene (25.26g) and dissolved in 1.4 L of dichloromethane. m-CBPA (85g) is added in portions over a 20-30 minute period. The mixture is heated to reflux for about 5 hours and the reaction monitored by HPLC. The mixture is cooled to room temperature and 950 mL of sodium hydrogen sulfite is added. The solution is stirred for 15 minutes. The aqueous layer is removed and the organic phase is washed with aqueous sodium bicarbonate (-2x950 mL). The organic phase is separated, dried over magnesium sulfate and concentrated to give the sulfone compound as a greenish solid (26.56g crude). Purification of the sulfone is conducted as follows: the crude material is first recrystallized from EtOH/hexanes to give 15.64g of product (59% recovery). A second crop is recrystallized from EtOH to give 2.26g of product, improving the recovery to 68%.

A flask is charged with 6-methoxybenzothiophene sulfone (6.31g) and dissolved in 115 mL of chloroform. Bromine (dissolved in 10 mL of chloroform) is added dropwise over the course of 10 minutes. After about 4.5 hours TLC reveals consumption of starting material. The reaction is quenched by addition of triethylamine (5 mL). After stirring at room temperature for about 30 minutes, 450 mL of H₂O is added. The organic layer is separated and washed with 450 mL of brine, dried over magnesium sulfate and concentrated (11.50 g crude). After charcoal treatment, 7.73g of 6-methoxy-2-bromobenzothiophene sulfone is isolated. The brominated sulfone is purified according to the following protocol: 50 mL of EtOH is added to the crude material and the mixture is heated to reflux for 45 minutes and brought to room temperature. After cooling in an ice bath for 30 minutes the solid is filtered through a glass frit and washed with cold EtOH ( ∼3x20 mL). 6-Methoxy-2-bromobenzothiophene sulfone (6.29 g) is recovered as a first crop (81%).

A flask is charged with 6-methoxy-2-bromobenzothiophene sulfone (8.05g) and 100 mL of chloroform is added. Bromine (7.0 g, 1.5 eq.) in 50 mL of chloroform is added via addition funnel over the course of 20-30 minutes. After stirring for about 13 hours HPLC shows 3.5% starting material. 10 mL of triethylamine is added. After stirring at room temperature for 4 hours, 450 mL of H₂O is added and the organic layer is extracted. The organic layer is washed with 450 mL of brine and subsequently dried over magnesium sulfate and concentrated to give 6-methoxy-2,3-dibromobenzothiophene sulfone as a brownish solid. The dibrominated sulfone compound is purified according to the following protocol: 70 mL of EtOH is added to the compound and the mixture is heated to reflux for 45 minutes. The hot solution is cooled to room temperature and placed in an ice bath for 30 minutes. The crystals are filtered through a glass frit and washed with several portions of cold EtOH (- 3x20 mL) to give the dibrominated product (8.18 g) in 79% overall yield.

A flask is charged with 6-methoxy-2,3-dibromobenzothiophene sulfone (11.42 g) and 311 mL of THF is added. The temperature is reduced to 5 °C and the mixture is stirred at this temperature for about 15 minutes. Solid 4-(2-piperidin-1-yl-ethoxy)-phenol (7.84 g, 1.1 eq.) is added, followed by cesium carbonate (31.5 g, 3.0 eq.). The mixture is stirred for 15 minutes and then slowly brought to room temperature. After overnight stirring (13 hours), TLC reveals near consumption of starting material. 200 mL of H₂O is added followed by extraction with ethyl acetate ( 5x500 mL). The organic layers are combined and dried over magnesium sulfate. Solvent is removed under rotary evaporator to give 6-methoxy-3-[4-(2-piperidin-1-yl-ethoxy)-phenoxy]-2-bromobenzo[b]thiophene sulfone (14.47g crude). The solid is purified by the following protocol: 100 mL of EtOH is added to a flask containing the solid and heated to reflux for 1 hour. The slurry is then allowed to cool to room temperature. The mixture is cooled in an ice bath for about 30-45 minutes. The solid is filtered and washed with cold EtOH. Based on the amount of initial crude material, the recovery as a first crop is about 83% (12.0 g).

6-Methoxy-2-bromo-3-[4-(2-piperidin-1-yl-ethoxy)-phenoxy]-2-bromobenzo[b]thiophene sulfone (150 g, 303 mmol) and 15 g of 10% Pd-C are combined with 1400 mL of THF. EtOH (1400 mL) is added and the mixture rapidly stirred while the vessel is evacuated and purged with hydrogen several times. The reaction is stirred under hydrogen overnight at room temperature. Purge the reaction vessel with nitrogen, add Celite, stir, filter and rinse several times with MeOH. Remove the volatiles using a rotary evaporator, add Et₂O and concentrate to yield 6-methoxy-3-[4-(2-piperidin-1-yl-ethoxy)-phenoxy] benzo[b]thiophene sulfone. The product is purified by recrystallization from EtOH. This material is dissolved in methylene chloride and washed twice with saturated NaHCO₃, brine, then dried, filtered and concentrate to yield 112 g (89%) of 6-methoxy-3-[4-(2-piperidin-1-yl-ethoxy)-phenoxy] benzo[b]thiophene sulfone.

Dissolve 6-methoxy-3-[4-(2-piperidin-1-yl-ethoxy)-phenoxy] benzo[b]thiophene sulfone in 1.5 L of dioxane and add diisobutylaluminum hydride (1.617 L of a 1M solution in THF). Heat the solution to reflux for about 4 hours. Cool the solution to room temperature, slowly add 1L of EtOAc, carefully transfer to a 12L sep funnel containing 4L of 10% Rochelle salt (Na-K tartrate). Continued to add the rest of the reaction mixture slowly. Add 3L of EtOAc, continue to stir until the mixture cools down. Add solid NaCl, stir and allow to settle overnight. Separate layers, and wash the organic layer with water (2x), then brine, dry over Na₂SO₄, filter and concentrate to yield 105 g. Purify by flash chromatography (2 kg of silica gel, 1% → 5% MeOH/CH2Cl2) to yield 92.3 g (89%) of 6-methoxy-3-[4-(2-piperidin-1-yl-ethoxy)-phenoxy] benzo[b]thiophene.

Dissolve 6-methoxy-3-[4-(2-piperidin-1-yl-ethoxy)-phenoxy] benzo[b]thiophene in CH₂Cl₂ (950 mL). Add 13.37 mL of Br₂ in CH₂Cl₂ (50 mL) slowly. Allow the dark solution to stir for about 15 minutes at room temperature. Pour the mixture into 500 mL of a 10% aqueous Na₂S₂O₃ solution, separate and wash again with an additional 500 mL of Na₂S₂O₃ solution. Wash with saturated NaHCO₃ (1 x 500 mL, 1 x 300 mL), then brine. Dry over Na₂SO₄, filter and concentrate to yield 105 g of a dark oil. Purify by silica gel chromatography (3 kg of silica gel, 1 → 4% 2M NH3 in MeOH/CH₂Cl₂) to yield 96.25 g (88%) of the free base of title compound. Dissolve the residue in -500 mL of Et₂O and filter. Form the HCl salt by adding 104 mL of 2M HCl/Et₂O slowly to the rapidly stirring solution. Filter and wash with Et₂O 2x and dry to yield 99 g (96%) of the title compound.

### Example 16

### 2-(4-Methanesulfonyl-cylohex-1-enyl)-3-[4-(2-piperidin-1-yl-ethoxy)-phenoxy]-benzo[b]thiophen-6-ol hydrochloride

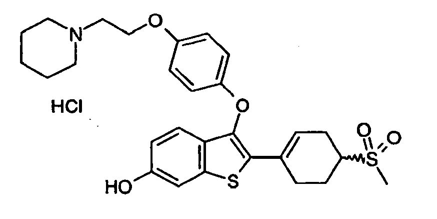

Combine 6-methoxy-3-[4-(2-piperidin-1-yl-ethoxy)-phenoxy]-2-bromobenzo[b]thiophene (0.44 g, 0.88 mmol), (+/-)-2-(4-methanesulfonyl-cyclohex-1-enyl)-4,4,5,5-tetramethyl-[1,3,2]dioxaborolane (0.50 g, 1.76 mmol) and tetrakis(triphenylphosphene)palladium(0) (0.10 g, 0.09 mmol) in 1,4-dioxane (20 mL) and bubble nitrogen through the solution for 15 minutes. Add 2M aqueous sodium carbonate solution (0.93 mL, 1.85 mmol) and heat the reaction mixture to 100°C for 5 hours. Cool to ambient temperature and stir for 64 hours. Partition between saturated aqueous ammonium chloride solution (50 mL) and ethyl acetate (100 mL). Separate the organic layer and wash with brine solution (30 mL), dry over magnesium sulfate, filter and concentrate in vacuo. Purify the residue by column chromatography using a silica gel column eluting with 1:1 hexane:ethyl acetate + 2% 7M ammonia/methanol to obtain 200 mg (42%) 1-(2-{4-[2-(4-methanesulfonyl-cyclohex-1-enyl)-6-methoxy-benzo[b]thiophen-3-yloxy]-phenoxy}-ethyl)-piperidine.

Dissolve 1-(2-{4-[2-(4-methanesulfonyl-cylohex-1-enyl)-6-methoxy-benzo[b]thiophen-3-yloxy]-phenoxy}-ethyl)-piperidine (200 mg, 0.37 mmol) in CH₂Cl₂ (10 mL) and treat with hydrogen chloride (0.5 mL, 1.0 M in ether) and stir the reaction mixture for 10 minutes. Concentrate in vacuo to obtain 200 mg (94%) 1-(2-{4-[2-(4-methanesulfonyl-cyclohex-1-enyl)-6-methoxy-benzo[b]thiophen-3-yloxy]-phenoxy}-ethyl)-piperidine hydrochloride.

Dissolve 1-(2-{4-[2-(4-methanesulfonyl-cyclohex-1-enyl)-6-methoxy-benzo[b]thiophen-3-yloxy]-phenoxy}-ethyl)-piperidine hydrochloride (200 mg, 0.35 mmol) in CH₂Cl₂ (10 mL) and 2-methyl-1-butene (2 mL). Cool the solution to 0°C and add BBr₃ (1.21 mL, 1.21 mmol, 1M in CH₂Cl₂). Stir at 0°C for 30 minutes, then warm to ambient temperature and stir another 3 hours. Partition between CH₂Cl₂ (100 mL) and saturated aqueous sodium bicarbonate solution (20 mL). Separate the organic layer and wash with brine solution (30 mL), dry over magnesium sulfate, filter and concentrate in vacuo. Purify the residue by column chromatography using a silica gel column eluting with 1:1 hexane:ethyl acetate + 2% 7M ammonia/methanol to obtain 75 mg (42%) of 2-(4-methanesulfonyl-cyclohex-1-enyl)-3-[4-(2-piperidin-1-yl-ethoxy)-phenoxy]-benzo[b]thiophen-6-ol: mass spectrum (ion spray): m/z = 527.8 (M+H).

Dissolve 2-(4-methanesulfonyl-cyclohex-1-enyl)-3-[4-(2-piperidin-1-yl-ethoxy)-phenoxy]-benzo[b]thiophen-6-ol (0.075g, 0.14 mmol) in CH₂Cl₂ (10 mL) and treat with hydrogen chloride (0.25 mL, 1.0 M in ether) and stir the reaction mixture for 10 minutes. Concentrate in vacuo to obtain 80 mg (99%) of the title compound: mass spectrum (ion spray): m/z = 527.8 (M+H-HCl).

### Formulation

Because the free base form of a compound of formula I contains a basic moiety (*i*.*e*., amino), said compound may be formulated as a pharmaceutical acid addition salt, *e*.*g*., as the hydrochloride salt or as a salt described in "Handbook of Pharmaceutical Salts: Properties, Selection and Use", Weinheim, New York: VHCA; Wiley-VCH, 2002.

The present pharmaceutical compositions are prepared by known procedures using well-known and readily available ingredients. In making the formulations of the present invention, the active ingredient (formula I compound) will usually be mixed with a carrier, or diluted by a carrier, or enclosed within a carrier which may be in the form of a capsule, sachet, paper or other container. When the carrier serves as a diluent, it may be a solid, semisolid or liquid material which acts as a vehicle, excipient or medium for the active ingredient.

Some examples of suitable carriers, excipients, and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents.

### Biological Assays

*Estrogen Receptor Binding Assay*: Representative compounds of the present invention are screened for binding affinity to both estrogen receptor types (ERα and ERβ). This competition binding assay measures the compound's ability to displace ³H-estradiol and generates IC₅₀ and Kᵢ values for both receptor types.

This competition binding assay is run in a buffer containing 50mM Hepes, pH 7.5, 1.5mM EDTA, 150mM NaCl, 10% glycerol, 1mg/mL ovalbumin and 5mM DTT, using 0.025 µCi per well ³H-Estradiol(NEN #NET517 at 118 Ci/mmol, 1 mCi/mL), 10 ng/well ERAlpha or ERbeta receptor (PanVera). A compound of the present invention is added at 10 different concentrations. Non-specific binding is determined in the presence of 1uM of 17-B Estradiol. The binding reaction (140 µl) is incubated for 4 hours at room temperature, then 70 µl of cold DCC buffer is added to each reaction (DCC buffer contains per 50 mL of assay buffer, 750 mg of charcoal (Sigma) and 250 mg of dextran (Pharmacia)). Plates are mixed 8 minutes on an orbital shaker at 4°C. Plates are then centrifuged at 3,000 rpm at 4°C for 10 minutes. An aliquot of 120 µl of the mix is transferred to another 96-well, white flat bottom plate (Costar) and 175 µl of Wallac Optiphase "Hisafe 3" scintillation fluid is added to each well. Plates are sealed and shaken vigorously on an orbital shaker. After an incubation of 2.5 hours, the plates are read in a Wallac Microbeta counter. The data is used to calculate an IC₅₀ and % Inhibition at 10µM. The K_{d} for ³H-Estradiol is determined by saturation binding to ER alpha and ER beta receptors. The IC₅₀ values for test compounds are converted to Kᵢ using Cheng-Prusoff equation and the K_{d} determined by saturation binding assay.

*Ishikawa Cell Proliferation Assay*: This assay measures cell proliferation (using an alkaline phosphatase readout) in both an agonist mode in the presence of a compound of the present invention alone, and in an antagonist mode in which the ability of a compound of the present invention to block estradiol stimulation of growth is measured.

Ishikawa human endometrial tumor cells are maintained in MEM (minimum essential medium, with Earle's salts and L-Glutamine, Gibco BRL, Gaithersburg, MD), supplemented with 10% fetal bovine serum (FBS) (V/V), (Gibco BRL). One day prior to assay, growth media is changed to assay medium, DMEM/F-12 (3:1) (Dulbecco's Modified Eagle Medium: Nutrient Mixture F-12, 3:1 Mixture, phenol red-free, Gibco BRL) supplemented with 5% dextran coated charcoal stripped fetal bovine serum (DCC-FBS) (Hyclone, Logen, UT), L-Glutamine (2mM), MEM sodium pyruvate (1 mM), HEPES (N-[2-hydroxyethyl]piperazine-N' - [2-ethanesulfonic acid] 2 mM) all from Gibco BRL). After an overnight incubation, Ishikawa cells are rinsed with Dulbecco's Phosphate Buffered Saline (1X) (D-PBS) without Ca⁺² and Mg⁺² (Gibco BRL), and trypsinized by a 3 minute incubation with 0.25% Trypsin/EDTA, phenol red-free (Gibco BRL). Cells are resuspended in assay medium and adjusted to 250,000 cells/mL. Approximately 25,000 cells in a 100ul media are added to flat-bottom 96 wells microculture plates (Costar 3596) and incubated at 37°C in a 5% CO₂ humidified incubator for 24 hours. The next day, serial dilutions of compounds are prepared in assay medium (at 6 times the final concentration in the assay). The assay is run in dual mode, agonist and antagonist modes.

For the agonist mode, plates receive 25 µl/well of assay medium followed by 25 µl/well of a diluted compound of the present invention (at 6x the final concentrations). For the antagonist mode, plates receive 25 µl/well of 6 nM E₂ (β-Estradiol, Sigma, St. Louis, MO) followed by 25 µl/well of a diluted compound of the present invention (at 6x the final concentrations). After an additional 48-hour incubation at 37°C in a 5% CO₂ humidified incubator, media is aspirated from wells and 100 µl fresh assay medium is added to each microculture. Serial dilutions of compounds are prepared and added to the cells as described above. After an additional 72 hour incubation at 37°C in a 5% CO₂ humidified incubator, the assay is quenched by removing media and rinsing plates twice in Dulbecco's Phosphate Buffered Saline (1X) (D-PBS) (Gibco BRL). The plates are dried for 5 minutes and frozen at -70°C for at least 1 hour. The plates are then removed from the freezer and allowed to thaw at room temperature. To each well, 100 µl of 1-Step™ PNPP (Pierce Chemical Company, Rockford, IL) is added. After a 20-minute incubation, plates are read on a spectophotometer at 405nm.

The data is fitted to a linear interpolation to derive EC₅₀ (for agonist mode) or IC₅₀ (for antagonist mode) values. For the antagonist mode, a % efficacy for each compound is calculated versus E2 (1nM) alone. For the agonist mode, a % efficacy for each compound is calculated versus the response to tamoxifen.

In the agonist mode, the compounds of Examples 2-5, 9, 10 12 and 13 were tested and were found to be less stimulatory than tamoxifen. For example, the compound of Example 9 had a relative % efficacy of 19%. In the antagonist mode, these same compounds inhibited greater than at least 80% of the 1nM estradiol response. For example, the compound of Example 9 had an IC₅₀ of 7.2 nM and a % efficacy of 97.9%.

*MCF-7 Proliferation Assay*: The MCF-7 cell line is derived from a human breast adenocarcinoma and is used as an indicator of potential antiproliferative activity in breast epithelium. MCF-7 breast adenocarcinoma cells (ATCC HTB 22) are maintained in MEM (minimal essential medium, phenol red-free, Gibco BRL) supplemented with 10% fetal bovine serum (FBS) (V/V), L-glutamine (2 mM), sodium pyruvate (1 mM), HEPES ((N-[2-hydroxyethyl]piperazine-N'-[2-ethanesulfonic acid] 10 mM}, non-essential amino acids(0.1mM)and Penicillin Streptomycin(1X). Seven days prior to assay, MCF-7 cells are switched to assay media which is the same as maintenance medium except supplemented with 10% dextran-coated charcoal-stripped fetal bovine serum (DCC-FBS) assay medium in place of 10% FBS. MCF-7 cells are removed from flasks using 10X Trypsin EDTA (phenol red free, Gibco BRL) and diluted to 1X in (Ca++/Mg++ free HBSS (phenol red-free). Cells are adjusted to 80,000 cells/mL in assay medium. Approximately 8,000 cells (100 µl) are added to each well in 96 well Cytostar T scintillation plates (Amersham) and incubated at 37°C in a 5% CO₂ humidified incubator for 24 hours to allow cell adherence and equilibration after transfer.

Serial dilutions of a compound of the present invention are prepared in assay medium at 4x the final desired concentration). A 50 µl aliquot of test compound dilutions (at 4x the final assay concentration) is transferred to duplicate wells followed by 50 µl assay medium for the agonist mode or 50 µl of 40pM of E2 for the antagonist mode to a final volume of 200 µl. For each of the agonist plates, a basal level (media) and a maximum stimulated level (with 1µM E2) is determined. For each of the antagonist plates, a basal level (media) and an E2 (10pM) alone control is determined. After an additional 48 hours at 37°C in a 5% CO₂ humidified incubator, 20µl of assay medium containing 0.01 µCi of ¹⁴C-thymidine (52 mCi/mmol, 50 µCi/ul, Amersham) is added to each well. The plates are incubated overnight in the same incubator and then counted on the Wallac Microbeta counter. The data is averaged to calculate an IC₅₀ and % inhibition @ 1µM for the antagonist mode. For the agonist mode, an EC₅₀ and percent of maximum E2 stimulation and concentration of maximum stimulation is calculated.

*3-Day Rat Uterus Antagonist Assay*: This model for uterine antagonism utilizes immature (3 week old) female rats that are highly sensitive to estrogenic stimulation of the uterus given that their circulating estrogen levels are prepubertal. The uteri from immature rats are fully responsive to exogenous estrogen, yet are quiescent in the absence of exogenous estrogen. Administration of exogenous estrogen to immature rats produces a reliable elevation of uterine weight, which can be used to study uterine antagonist effects. The rats are treated with both estradiol and 4 different concentrations of a compound of the present invention for 3 days and then uterine wet weights are measured.

Nineteen to twenty-one day old (or 45-50g) female rats are orally treated with E2 (0.1 mg/kg, a maximal stimulatory estrogenic stimulus for reliably increasing uterine weight) and 10, 1.0, 0.1 and 0.01mg/kg test compound for 3 days, 6 rats per group. Test compounds are dissolved in 20% β-hydroxycyclodextrin and administered by oral gavage in a volume of 0.2 mL daily (15 min. prior to the ethynyl estradiol gavage). A vehicle control, E2 alone and E2 + raloxifene are also done as controls. The animals are fasted overnight following the final dose. On the following morning, the animals are weighed, then euthanized (by carbon dioxide asphyxiation) and the uteri rapidly collected (via a mid-line ventral incision) and weighed.

Uterine weight/body weight ratios (UWR) are calculated for each animal. The percent inhibition of the estrogen-induced response is then calculated by the following formula: percent inhibition = 100 x (UWR_{estrogen} - UWR_{test compound}/UWR_{estrogen} - UWR_{control}). ED50 values are derived from a semi-log regression analysis of the linear aspect of the dose response curve. Both the UWR data and the percent inhibition data are statistically analyzed by one way analysis of variance (ANOVA) with post-hoc testing by Fisher's PLSD when indicated by a p ≤ 0.05. Statistical analyses are performed using the Statview® 4.0 software package.

The compounds of Examples 2, 3, 9 and 10 were tested in the above assay and were found to inhibit the estrogen-induced response when administered at 1.0 mg/kg. For example, the compound of Example 9 had an ED₅₀ of 0.17 mpk and a % antagonism of 72.6%.

*4-Day OVX Rat Uterine Agonist Assay:* In order to assure that a test compound does not have any partial uterine agonist activity, compounds are administered to mature, ovariectomized rats.

Seventy-five day old rats are ovariectomized and treatment is started 14 days later when circulating estradiol levels have reached minimal levels. After 4 days of treatment with 3 doses of a compound of the present invention, (6 rats per group) body weight, uterine wet weight and uterine eosinophil peroxidase (EPO) activity are measured. Cholesterol levels are also measured to compare relative ability to lower cholesterol with other SERMs. If there is any question of uterine stimulation, histological examination will determine epithelial cell height.

*10-Day Rat Hormone (Ovarian Stimulation) Screen:* An initial, first screen for ovarian toxicity is conducted using a 10-day rat hormone study to measure estradiol and luteinizing hormone levels after compound administration. This screen is conducted by administering compound by oral gavage for 10 days to mature (9-10 week old) F344 female rats. Trunk blood is collected by rapid decapitation for evaluation of LH and estradiol levels approximately 2 hours after the 10^{th} dose. Serum, obtained by centrifugation, is removed and stored frozen below -60°C until assayed. Serum levels of LH and estradiol are measured using radioimmunoassay (RIA) methods.

Rat LH primary antibody and reference preparations (rat LH:RP-3) are obtained from Dr. A. F. Parlow, Director, Pituitary Hormones and Antisera Center, Harbor-UCLA Medical Center, Torrance, CA. The LH assay upper limits of detection are 30 ng/mL and the lower limits of detection are 0.1 ng/mL for the 100 µl samples.

E2 Clinical Assays. DiaSorin s.r.l., Saluggia (Vercelli), Italy. The upper limit of detection is 1000 pg/mL and the lower limit of detection is 5 pg/mL. The compound of Example 2 was tested in the above assay and did not significantly elevate circulating estradiol or LH levels.

*35-Day Ovary-Intact Rat Bone Assay*: While previous SERMs, including raloxifene have shown efficacy in preventing bone loss in OVX rats, the possibility of interference with estrogen-regulated turnover in ovary-intact rats needs to be addressed.

This assay is done in mature rats with concentrations based on the demonstrated efficacy in the 3-day assay. Generally, at least three concentrations are chosen based on multiples of the ED50 generated therein. These multiples are generally 1x, 10x and 30x the ED50. A compound of the present invention is administered to an OVX rat for 35 days and is compared to control, ovariectomized, and/or GnRH-administered rats. Femurs, tibiae, uteri, ovaries and serum are taken for further analyses. DEXA (Dual Energy X-ray Absorptivity), CT (Computed Tomography) and histologic analysis are done on the long bones to assess any changes. CT scans of the distal femur are done to calculate BMD (bone mineral density), cross sectional area and BMC (bone mineral content). Bone strength measurements (load to failure) may also be done to determine consequences of any bone mass or material changes. Uterine and ovarian histology are examined to confirm long term dosing effects of uterine efficacy and potential ovarian stimulation. The serum is analyzed for LH and E2 levels as a possible indicator of ovarian effects.

### Utilities

The diseases, disorders or conditions for which a compound of formula I is useful in treating include, but are not limited to, (1) uterine cancer; (2) endometriosis; (3) uterine leiomyoma/leiomyomata; (4) post-menopausal osteoporosis, *i.e.*, osteoporosis caused by the loss of bone that results from a lack of endogenous estrogen such as occurs in a woman following cessation of menstration due to natural, surgical, or other processes; and (5) estrogen receptor postive (ER+) breast cancer, particularly the prevention thereof. Treatment of uterine leiomyoma/leiomyomata as described herein, also contemplates the reduction of the occurrence or severity of the associated symptoms such as pain, urinary frequency, and uterine bleeding.

### Dose

The specific dose administered is determined by the particular circumstances surrounding each situation. These circumstances include, the route of administration, the prior medical history of the recipient, the pathological condition or symptom being treated, the severity of the condition/symptom being treated, and the age of the recipient. The recipient patient's physician should determine the therapeutic dose administered in light of the relevant circumstances.

Generally, an effective minimum daily dose of a compound of formula I,will exceed about 5 mg. Typically, an effective maximum daily dose will not exceed about 350 mg. The exact dose may be determined, in accordance with the standard practice in the medical arts of "dose titrating" the recipient; that is, initially administering a low dose of the compound, and gradually increasing the does until the desired therapeutic effect is observed.

## Claims

1. A compound of formula I: 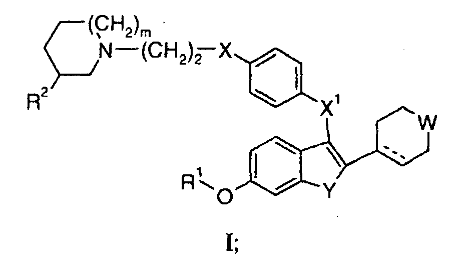 wherein:
m is 0, 1 or 2;
R¹ is H, SO₂(n-C₄-C₆ alkyl) or COR³;
R² is H or methyl provided that if m is 1 or 2, then R² must be H and that if m is 0, then R² must be methyl;
W is CHSO₂R⁴ or SO₂;
X is O or NR⁵;
X¹ is O, CH₂, or CO;
Y is S or CH=CH;
the dashed line ( ⁻⁻⁻ ) represents an optional double bond;
R³ is C₁-C₆ alkyl, C₁-C₆ alkoxy, NR⁶R⁷, phenoxy, or phenyl optionally substituted with halo;
R⁴ is C₁-C₆ alkyl, C₁-C₆ alkoxy, NR⁸R⁹, CF₃ or CH₂CF₃;
R⁵ is H or C₁-C₆ alkyl
R⁶, R⁷ and R⁸ are independently H, C₁-C₆ alkyl or phenyl; and
R⁹ is C₁-C₆ alkyl or phenyl; or a pharmaceutical acid addition salt thereof;
wherein "C₁-C₆ alkyl" represents a straight, branched or cyclic hydrocarbon.

2. The compound of claim 1 wherein R¹ is H or COR³, R³ is C₁-C₄ alkyl, NHCH₃ or phenyl, X and X¹ are O and m is 1 or 2.

3. The compound of claim 1 or 2 wherein R¹ is H and m is 1.

4. The compound of any one of claims 1-3 wherein R⁴ is C₁-C₄ alkyl, CF₃ or NR⁸R⁹ and R⁸ is H or C₁-C₄ alkyl, R⁹ is C₁-C₄ alkyl, W is CHSO₂R⁴ and Y is CH=CH.

5. The compound of any one of claims 1-3 wherein R⁴ is C₁-C₄ alkyl, CF₃ or NR⁸R⁹ and R⁸ is H or C₁-C₄ alkyl, R⁹ is C₁-C₄ alkyl, Y is CH=CH, W is SO₂ and the optional double bond is not present.

6. The compound of any one of claims 1-5 wherein R⁴ is methyl, ethyl, cyclopropyl, CF₃, NHCH₃ or N(CH₃)₂.

7. The compound of claim 1 selected from the group consisting of: 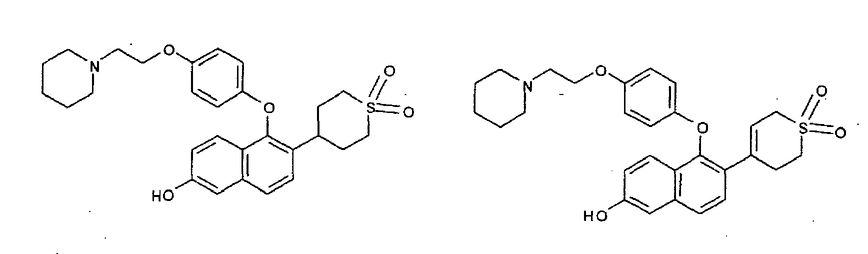 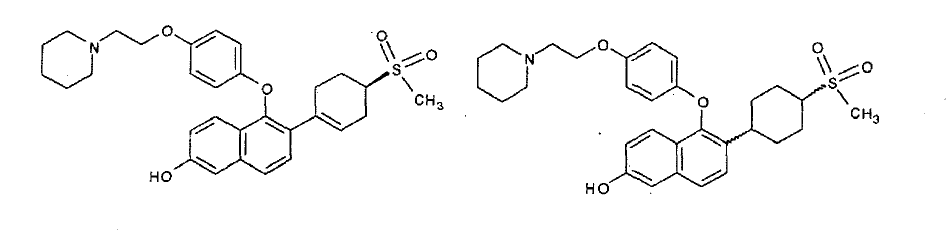 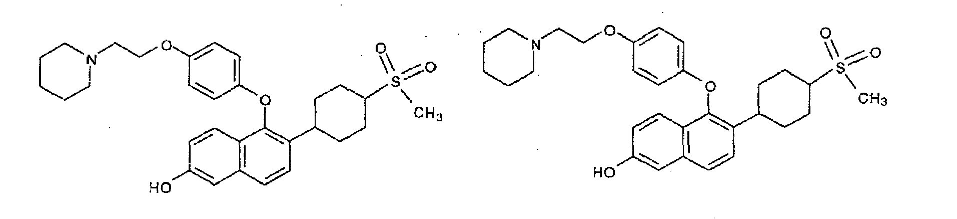 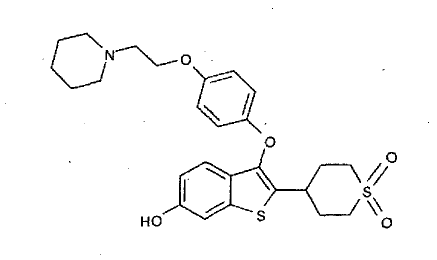 or a pharmaceutical acid addition salt thereof.

8. A compound of any one of claims 1-7 for use as a medicament.

9. Use of a compound of any one of claims 1-7 for the manufacture of a medicament for the treatment of endometriosis and/or uterine leiomyoma.

10. A compound of formula II: 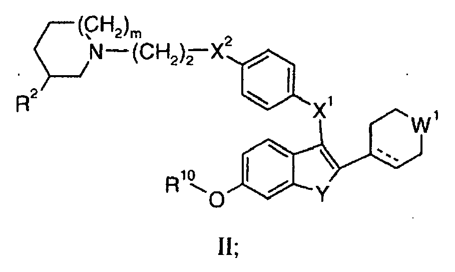 wherein:
m is 0, 1 or 2;
R² is H or methyl provided that if m is I or 2, then R² must be H and that if m is 0, then R² must be methyl;
R¹⁰ is H, C₁-C₆ alkyl, benzyl, SO₂CH₃, SO₂(n-C₄-C₆ alkyl) or COR⁴;
W¹ is CHS(O)ₙR⁴ or S(O)ₙ;
X¹ is O, CH₂, or CO;
X² is O or NR¹¹;
Y is S or CH=CH;
the dashed line ( ⁻⁻⁻ ) represents an optional double bond;
n is 0, 1 or 2;
R³ is OH, C₁-C₆ alkyl, C₁-C₆ alkoxy, NR⁶R⁷, phenoxy, or phenyl optionally substituted with halo;
R⁴ is C₁-C₆ alkyl, C₁-C₆ alkoxy, NR⁸R⁹, CF₃ or CH₂CF₃;
R⁶, R⁷ and R⁸ are independently H, C₁-C₆ alkyl or phenyl;
R⁹ is C₁-C₆ alkyl or phenyl; and
R¹¹ is H, C₁-C₆ alkyl or CO₂(C₁-C₆ alkyl); provided that if n is 2, then R¹⁰ is C₁-C₆ alkyl, SO₂CH₃ or benzyl or R¹¹ is CO₂(C₁-C₆ alkyl); or an acid addition salt thereof.

11. The compound of claim 10 wherein m is 1, R⁴ is methyl, ethyl, cyclopropyl, CF₃, NHCH₃ or N(CH₃)₂, R¹⁰ is SO₂CH₃, benzyl or methyl and W¹ is CHSOₙR⁴.

## Patentansprüche

1. Verbindung der Formel I: 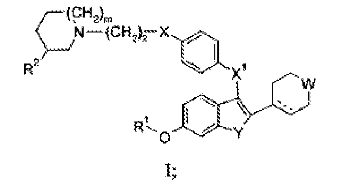 worin m für 0, 1 oder 2 steht;
R¹ für H, SO₂(n-C₄-C₆-Alkyl) oder COR³ steht;
R² für H oder Methyl steht, vorausgesetzt, dass wenn m für 1 oder 2 steht, dann R² für H stehen muss und dass, wenn m für 0 steht, dann R² für Methyl stehen muss;
W für CHSO₂R⁴ oder SO₂ steht;
X für O oder NR⁵ steht;
X¹ für O, CH₂ oder CO steht;
Y für S oder CH=CH steht;
die gestrichelte Linie (⁻⁻⁻) für eine optionale Doppelbindung steht;
R³ für C₁-C₆-Alkyl, C₁-C₆-Alkoxy, NR⁶R⁷, Phenoxy oder Phenyl, das optional mit Halogen substituiert ist, steht;
R⁴ für C₁-C₆-Alkyl, C₁-C₆-Alkoxy, NR⁸R⁹, CF₃ oder CH₂CF₃ steht;
R⁵ für H oder C₁-C₆-Alkyl steht;
R⁶, R⁷ und R⁸ unabhängig voneinander für H, C₁-C₆-Alkyl oder Phenyl stehen; und
R⁹ für C₁-C₆-Alkyl oder Phenyl steht; oder ein pharmazeutisches Säureadditionssalz hiervon; worin der Ausdruck "C₁-C₆-Alkyl" für einen geradkettigen, verzweigten oder zyklischen Kohlenwasserstoff steht.

2. Verbindung nach Anspruch 1, worin R¹ für H oder COR³ steht, R³ für C₁-C₄-Alkyl, NHCH₃ oder Phenyl steht, X und X¹ für O stehen und m für 1 oder 2 steht.

3. Verbindung nach Anspruch 1 oder 2, worin R¹ für H steht und m für 1 steht.

4. Verbindung nach einem der Ansprüche 1 bis 3, worin R⁴ für C₁-C₄-Alkyl, CF₃ oder NR⁸R⁹ steht und R⁸ für H oder C₁-C₄-Alkyl steht, R⁹ für C₁-C₄-Alkyl steht, W für CHSO₂R⁴ steht und Y für CH=CH steht.

5. Verbindung nach einem der Ansprüche 1 bis 3, worin R⁴ für C₁-C₄-Alkyl, CF₃ oder NR⁸R⁹ steht und R⁸ für H oder C₁-C₄-Alkyl steht, R⁹ für C₁-C₄-Alkyl steht, Y für CH=CH steht, W für SO₂ steht und die optionale Doppelbindung nicht vorhanden ist.

6. Verbindung nach einem der Ansprüche 1 bis 5, worin R⁴ für Methyl, Ethyl, Cyclopropyl, CF₃, NHCH₃ oder N(CH₃)₂ steht.

7. Verbindung nach Anspruch 1, ausgewählt aus der Gruppe, die aus den folgenden Verbindungen besteht: 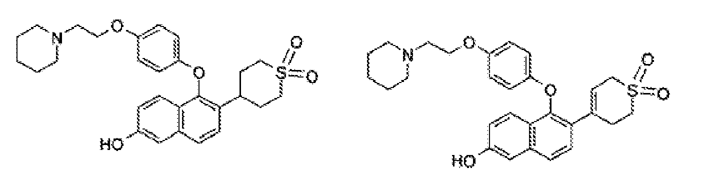 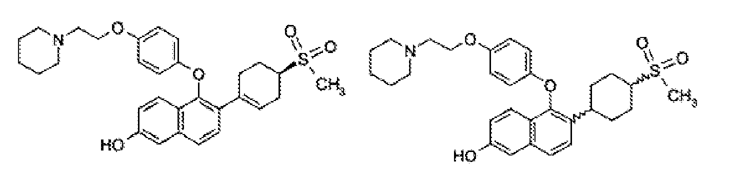 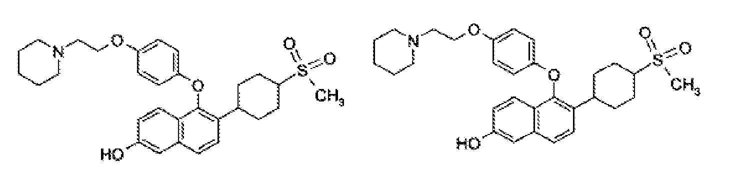 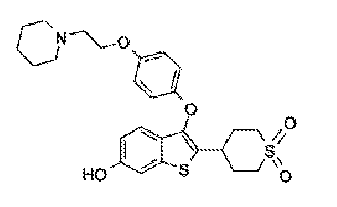 oder ein pharmazeutisches Säureadditionssalz hiervon.

8. Verbindung nach einem der Ansprüche 1 bis 7 zur Verwendung als Medikament.

9. Verwendung einer Verbindung nach einem der Ansprüche 1 bis 7 zur Herstellung eines Medikaments zur Behandlung von Endometriose und/oder Uterusleiomyom.

10. Verbindung der Formel II: 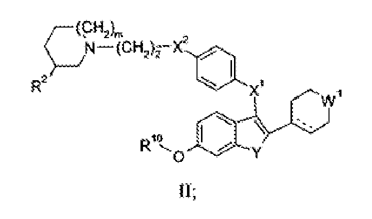 worin m für 0, 1 oder 2 steht;
R² für H oder Methyl steht, vorausgesetzt, dass, wenn m für 1 oder 2 steht, dann R² Wasserstoff bedeuten muss und dass, wenn m für 0 steht, R² Methyl bedeuten muss;
R¹⁰ für H, C₁-C₆-Alkyl, Benzyl, SO₂CH₃, SO₂(n-C₄-C₆-Alkyl) oder COR⁴ steht;
W¹ für CHS(O)ₙR⁴ oder S(O)ₙ steht;
X¹ für O, CH₂ oder CO steht;
X² für O oder NR¹¹ steht;
Y für S oder CH=CH steht;
die gestrichelte Linie (⁻⁻⁻) für eine optionale Doppelbindung steht;
n für 0, 1 oder 2 steht;
R³ für OH, C₁-C₆-Alkyl, C₁-C₆-Alkoxy, NR⁶R⁷, Phenoxy oder Phenyl, das optional mit Halogen substituiert ist steht;
R⁴ für C₁-C₆-Alkyl, C₁-C₆-Alkoxy, NR⁸R⁹, CF₃ oder CH₂CF₃ steht;
R⁶, R⁷ und R⁸ unabhängig voneinander für H, C₁-C₆-Alkyl oder Phenyl steht;
R⁹ für C₁-C₆-Alkyl oder Phenyl steht; und
R¹¹ für H, C₁-C₆-Alkyl oder CO₂(C₁-C₆-Alkyl) steht; wobei gilt, dass, wenn n für 2 steht, dann R¹⁰ für C₁-C₆-Alkyl, SO₂CH₃ oder Benzyl steht oder R¹¹ für CO₂(C₁-C₆-Alkyl) steht; oder ein Säureadditionssalz hiervon.

11. Verbindung nach Anspruch 10, worin m für 1 steht, R⁴ für Methyl, Ethyl, Cyclopropyl, CF₃, NHCH₃ oder N(CH₃)₂ steht, R¹⁰ für SO₂CH₃, Benzyl oder Methyl steht und W¹ für CHSOₙR⁴ steht.

## Revendications

1. Composé de formule 1 : 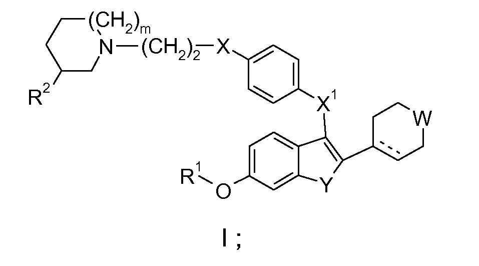 dans laquelle :
m vaut 0, 1 ou 2 ;
R¹ représente H, un groupe SO₂[n-(C₄-C₆)alkyle] ou COR³ ;
R² représente H ou un groupe méthyle sous réserve que lorsque m vaut 1 ou 2, R² est obligatoirement H et que lorsque m vaut 0, R² est obligatoirement un groupe méthyle ;
W représente CHSO₂R⁴ ou SO₂ ;
X représente O ou NR⁵;
X¹ représente O, CH₂, ou CO ;
Y représente S ou CH=CH ;
la ligne en pointillé ( ⁻⁻⁻ ) représente une double liaison facultative ;
R³ représente un groupe (C₁-C₆)alkyle, (C₁-C₆)alcoxy, NR⁶R⁷, phénoxy, ou phényle éventuellement substitué par un halogène ;
R⁴ représente un groupe (C₁-C₆)alkyle, (C₁-C₆)alcoxy, NR⁸R⁹, CF₃ ou CH₂CF₃;
R⁵ représente H ou un groupe (C₁-C₆)alkyle
R6, R⁷ et R⁸ représentent indépendamment H, un groupe (C₁-C₆)alkyle ou phényle ; et
R⁹ représente un groupe (C₁-C₆)alkyle ou phényle ; ou un sel d'addition d'acide pharmaceutique de celui-ci ;
où le groupe « (C₁-C₆)alkyle » représente un hydrocarbure linéaire, ramifié ou cyclique.

2. Composé selon la revendication 1, dans lequel R¹ représente H ou COR³, R³ représente un groupe (C₁-C₄)alkyle, NHCH₃ ou phényle, X et X¹ représentent O et m vaut 1 ou 2.

3. Composé selon la revendication 1 ou 2, dans lequel R¹ représente H et m vaut 1.

4. Composé selon l'une quelconque des revendications 1-3, dans lequel R⁴ représente un groupe (C₁-C₄)alkyle, CF₃ ou NR⁸R⁹ et R⁸ représente H ou un groupe (C₁-C₄)alkyle, R⁹ représente un groupe (C₁-C₄)alkyle, W représente CHSO₂R⁴ et Y représente CH=CH.

5. Composé selon l'une quelconque des revendications 1-3, dans lequel R⁴ représente un groupe (C₁-C₄)alkyle, CF₃ ou NR⁸R⁹ et R⁸ représente H ou un groupe (C₁-C₄)alkyle, R⁹ représente un groupe (C₁-C₄)alkyle, Y représente CH=CH, W représente SO₂ et la double liaison facultative n'est pas présente.

6. Composé selon l'une quelconque des revendications 1-5, dans lequel R⁴ représente un groupe méthyle, éthyle, cyclopropyle, CF₃, NHCH₃ ou N(CH3)2.

7. Composé selon la revendication 1 choisi dans le groupe constitué de : 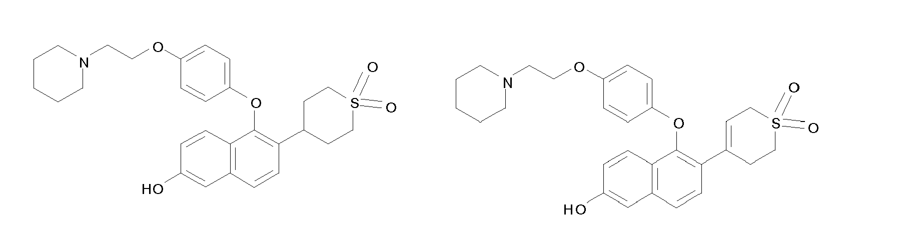 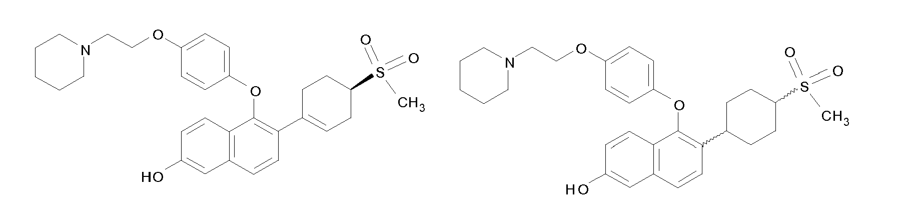 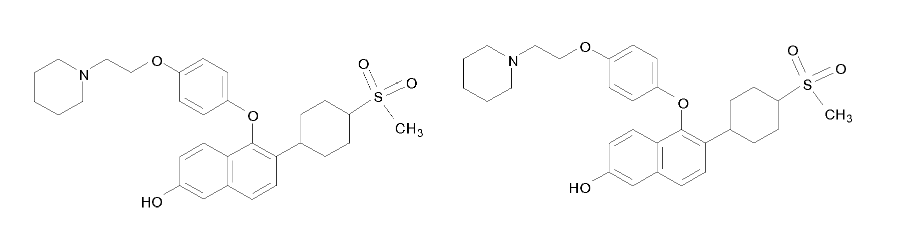 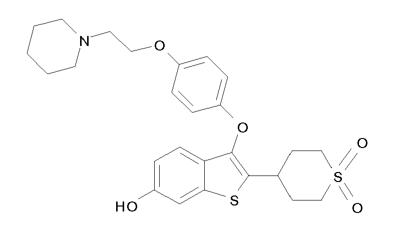 ou un sel d'addition d'acide pharmaceutique de celui-ci.

8. Composé selon l'une quelconque des revendications 1-7, destiné à être utilisé comme médicament.

9. Utilisation d'un composé selon l'une quelconque des revendications 1-7, pour la fabrication d'un médicament destiné au traitement de l'endométriose et/ou du fibromyome utérin.

10. Composé de formule II : 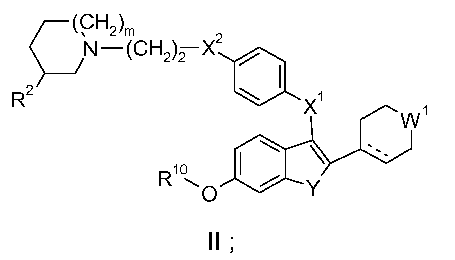 dans laquelle :
m vaut 0, 1 ou 2 ;
R² représente H ou un groupe méthyle sous réserve que lorsque m vaut 1 ou 2, R² est obligatoirement H et que lorsque m vaut 0, R² est obligatoirement un groupe méthyle ;
R¹⁰ représente H, un groupe (C₁-C₆)alkyle, benzyle, SO₂CH₃, SO₂[n-(C₄-C₆)alkyle] ou COR₄ ;
W¹ représente CHS(O)ₙR⁴ ou S(O)ₙ ;
X¹ représente O, CH₂, ou CO ;
X² représente O ou NR¹¹ ;
Y représente S ou CH=CH ;
la ligne en pointillé ( ⁻⁻⁻ ) représente une double liaison facultative ;
n vaut 0, 1 ou 2 ;
R³ représente OH, un groupe (C₁-C₆)alkyle, (C₁-C₆)alcoxy, NR⁶R⁷, phénoxy, ou phényle éventuellement substitué par un halogène ;
R⁴ représente un groupe (C₁-C₆)alkyle, (C₁-C₆)alcoxy, NR⁸R⁹, CF₃ ou CH₂CF₃;
R⁶, R⁷ et R⁸ représentent indépendamment H, un groupe (C₁-C₆)alkyle ou phényle ;
R⁹ représente un groupe (C₁-C₆)alkyle ou phényle ; et
R¹¹ représente H, un groupe (C₁-C₆)alkyle ou CO₂[(C₁-C₆)alkyle]; sous réserve que, lorsque n vaut 2, alors R¹⁰ représente un groupe (C₁-C₆)alkyle, SO₂CH₃ ou benzyle ou R¹¹ représente un groupe CO₂[(C₁-C₆)alkyle] ; ou un sel d'addition d'acide de celui-ci.

11. Composé selon la revendication 10, dans lequel m vaut 1, R⁴ représente un groupe méthyle, éthyle, cyclopropyle, CF₃, NHCH₃ ou N(CH₃)₂, R¹⁰ représente SO₂CH₃, un groupe benzyle ou méthyle et W¹ représente CHSOₙR⁴.
